# EUROPEAN PATENT APPLICATION

(11) **EP 0 976 747 A2**
(43) Date of publication of application: **02.02.2000**
(21) Application number: 99305923.7
(22) Date of filing: 26.07.1999
(51) Int. Cl.: C07D 401/04, C07D 401/14, A61K 31/445

(54) **Use of 5HT-1F receptor antagonists for treating anxiety disorders**

(30) Priority: 27.07.1998 US 94310 P
(71) Applicant: ELI LILLY AND COMPANY, Indianapolis, Indiana 46285 (US)
(72) Inventor: Phebus, Lee Alan, Fountaintown, Indiana 46130 (US); Sajdyk, Tammy Joy, Speedway, Indiana 46224 (US)
(74) Representative: Hudson, Christopher Mark

(57) **Abstract**

The present invention provides a method for the treatment of prevention of anxiety disorders which comprises administering to a mammal in need of such treatment a serotonin 5-HT_{1F} receptor antagonist.

## Description

Anxiety disorders represent the most prevalent type of psychiatric disorders in the United States. Anxiety disorders include panic disorder, obsessive-compulsive disorder, post-traumatic stress disorder, specific phobia, social phobia, and generalized anxiety disorder. All are characterized by uneasiness, a sense of fearfulness, and distress for no apparent reason. These disorders, if left untreated, reduce the quality of life and productivity of patients suffering from them. In the United States alone, more than 23 million people suffer from anxiety disorders. The cost to society from these disorders is staggering, estimated in 1990 at $46.6 billion in the United States alone in direct and indirect costs.

Currently available methods for treating anxiety disorders include behavioral therapy, cognitive therapy, and relaxation techniques. These methods typically take a considerable amount of time to achieve their desired effect. To increase the rate of recovery, these methods may be used in combination with one of a number of medications. Currently used medications include benzodiazepines, beta-blockers, buspirone, monoamine oxidase inhibitors, serotonin reuptake inhibitors, and tricyclic antidepressants, all of which have liabilities associated with their use. The benzodiazepines are potentially habit forming and can cause drowsiness; beta-blockers cannot be used if the patient has certain pre-existing medical conditions such as asthma, congestive heart failure, diabetes, vascular disease, hyperthyroidism, or angina pectoris; buspirone has a long induction period before its beneficial effects are realized; patients taking monoamine oxidase inhibitors are under strict dietary constraints and there is the potential for drug interactions, low blood pressure, moderate weight gain, reduced sexual response, and insomnia; the serotonin reuptake inhibitors can cause nausea, nervousness, and delayed ejaculation; and the tricyclic antidepressants can cause dry mouth, constipation, blurry vision, difficulty in urination, dizziness, low blood pressure, and moderate weight gain. New methods for treating anxiety disorders are needed which avoid or diminish the liabilities of current therapies.

Serotonin (5-HT) exhibits diverse physiological activity mediated by at least four receptor classes, the most heterogeneous of which appears to be 5-HT₁. A human gene which expresses a fifth 5-HT₁ subtype, named 5-HT_{1F}, was isolated by Kao and coworkers (*Proc. Natl. Acad. Sci. USA,* **90**, 408-412 (1993)). This 5-HT_{1F} receptor exhibits a pharmacological profile distinct from any serotonergic receptor yet described. While agonists of the 5-HT_{1F} receptor are known to be useful for the treatment and prevention of migraine pain (Audia, *et al.,* U.S. Patent No. 5,698,571), the utility of antagonists of the 5-HT_{1F} receptor for the treatment of anxiety disorders was heretofore unknown.

The present invention provides a method for the treatment or prevention of anxiety disorders which comprises administering to a mammal in need of such treatment an effective amount of a serotonin 5-HT_{1F} antagonist.

The present invention also provides a method for the prevention of anxiety disorders which comprises administering to a mammal susceptible to said disorders an effective amount of a serotonin 5-HT_{1F} antagonist.

The present invention provides a method for the treatment or prevention of anxiety disorders which relies on a novel mechanism of action. This method comprises treating a mammal suffering from or susceptible to anxiety disorders with a compound which is an antagonist at the 5-HT_{1F} receptor. This mechanism is operative in mammals and the preferred mammal is a human.

A further embodiment of this invention comprises the administration of a composition which exhibits 5-HT_{1F} antagonist activity. The composition may be composed of one or more agents which, individually or together, are antagonists of the 5-HT_{1F} receptor.

An especially preferred embodiment of this invention comprises the administration of a compound or composition which is a selective antagonist at the 5-HT_{1F} receptor relative to other serotonin receptors. While a compound or composition exhibiting any selectivity for the 5-HT_{1F} receptor relative to other serotonin receptors is preferred, a compound or composition exhibiting at least a 10 fold selectivity for the 5-HT_{1F} receptor relative to other serotonin receptors is more preferred. It is most preferred that the compound or composition exhibits at least a 100 fold selectivity for the 5-HT_{1F} receptor.

The term "5-HT_{1F} antagonist", as it is used in the description of this invention, is taken to mean a full antagonist at the 5-HT_{1F} receptor, a partial antagonist at the 5-HT_{1F} receptor, or an inverse agonist at the 5-HT_{1F} receptor.

The term "full antagonist", as it is used in this invention, is taken to mean a compound or composition with affinity for the 5-HT_{1F} receptor which also exhibits an intrinsic activity about 15% or less of the maximal effect of serotonin at the 5-HT_{1F} receptor. The use of a full antagonist is a preferred embodiment of the present invention.

The term "partial antagonist", as it is used in this invention, is taken to mean a compound or composition with affinity for the 5-HT_{1F} receptor which also exhibits an intrinsic activity greater than about 15% of the maximal effect of serotonin at the 5-HT_{1F} receptor and still exerts an anxiolytic effect at an acceptable dose. The use of a partial antagonist is another preferred embodiment of the present invention.

Compounds which exhibit negative intrinsic activity, known by the skilled artisan as inverse agonists, are also useful for the method of the present invention. Inverse agonists of the 5-HT_{1F} receptor bind to the 5-HT_{1F} receptor, block the effects of agonists at the 5-HT_{1F} receptor, and decrease the constitutive activity of the 5-HT_{1F} receptor.

The present invention provides a method for the treatment or prevention of anxiety disorders comprising administering to a mammal in need of such treatment an antagonist of the 5-HT_{1F} receptor. The principle of this invention is demonstrated by use of the 5-HT_{1F} antagonists of Formula I: 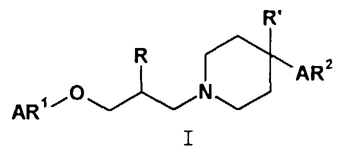 where:
R and R' are independently hydrogen or hydroxy;
AR¹ is phenyl, naphthyl, quinolinyl, isoquinolinyl, indanyl, 1,2,3,4-tetrahydronaphthyl, indolyl, N-(C₁-C₄ alkyl)indolyl, benzothiazolyl, benzothienyl, benzofuryl, 2,3-dihydrobenzothienyl, 2,3-dihydrobenzofuryl, julolidinyl, or dibenzofuryl, each optionally substituted with one or two substituents independently selected from the group consisting of C₁-C₆ alkyl, C₁-C₆ acyl, benzoyl, C₁-C₆ alkoxy, phenoxy, C₁-C₆ alkylthio, trifluoromethyl, trifluoromethoxy, or halo;
AR² is pyridin-3-yl, quinolin-3-yl, isoquinolin-4-yl, or quinoxalin-2-yl; and pharmaceutically acceptable acid addition salts thereof.

The general chemical terms used in the formulae above have their usual meanings. For example, the term "alkyl" includes such groups as methyl, ethyl, n-propyl, isopropyl, n-butyl, isobutyl, sec-butyl, tert-butyl, 1-pentyl, 2-pentyl, 3-pentyl, neopentyl, hexyl, and the like. The term "alkoxy" includes methoxy, ethoxy, isopropoxy, butoxy, tert-butoxy, hexyloxy, and the like. The term "alkylthio" includes methylthio, ethylthio, isopropylthio, butylthio, tert-butylthio, hexylthio, and the like. The term "acyl" includes formyl, acetyl, propanoyl, butanoyl, 2-methylpropanoyl, pentanoyl, and the like. The term "halo" includes fluoro, chloro, bromo and iodo.

The compounds of Formula I where R is hydroxy possess an asymmetric carbon labelled with an asterisk in the following formula: 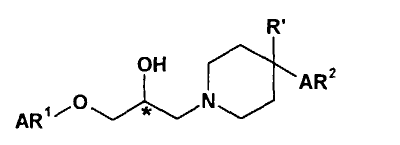 As such, each of the compounds of Formula exists not only as the racemate but as individual R- and S-enantiomers as well: 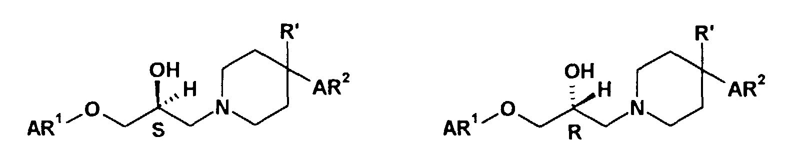 The compounds of Formula I include the individual R- and S-enantiomers, and mixtures thereof, including the racemates.

Since the compounds of Formula I are amines, they are basic in nature and accordingly react with any of a number of inorganic and organic acids to form pharmaceutically acceptable acid addition salts. Since many of the free amines of the compounds of this invention are oils or low melting amorphous solids, it is preferable to convert the free amines to their pharmaceutically acceptable acid addition salts for ease of handling and administration. Acids commonly employed to form such salts are inorganic acids such as hydrochloric acid, hydrobromic acid, hydroiodic acid, sulfuric acid, phosphoric acid, and the like, and organic acids, such as p-toluenesulfonic acid, methane-sulfonic acid, oxalic acid, p-bromophenylsulfonic acid, carbonic acid, succinic acid, citric acid, benzoic acid, acetic acid and the like. Examples of such pharmaceutically acceptable salts thus are the sulfate, pyrosulfate, bisulf-ate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, β-hydroxybutyrate, glycollate, tartrate, methanesulfonate, propanesulfonate, naphthalene-1-sulfonate, naphthalene-2-sulfonate, mandelate and the like.

The compounds of Formula I are prepared by standard synthetic organic chemistry methodology as illustrated in Synthetic Scheme I, where AR¹, AR², and R¹ are as previously defined.

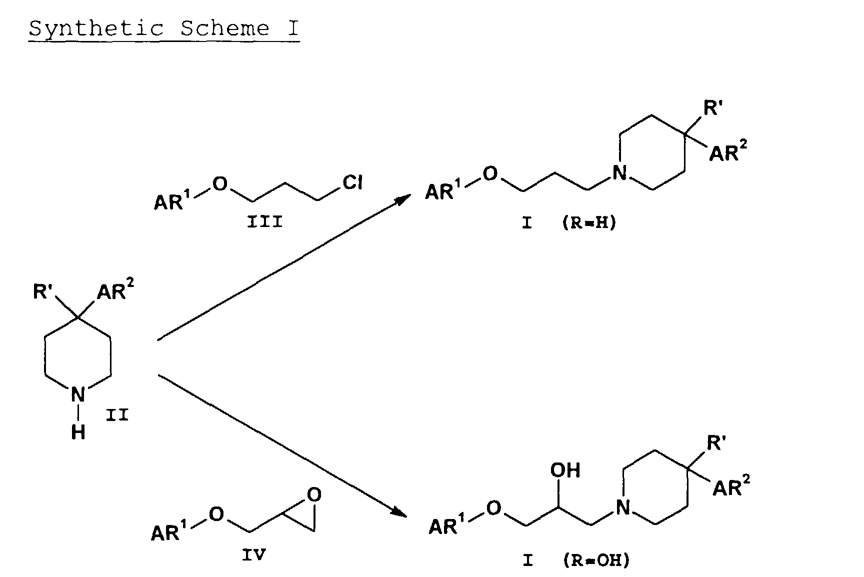

Compounds of the invention where R is hydrogen are prepared by reacting a substituted piperidine of Formula II with a 3-chloropropyl AR¹ ether of Formula III under standard alkylation conditions. The requisite piperidine and chloropropyl ether are combined in a mutual solvent, typically acetonitrile, with an appropriate base, typically potassium or sodium carbonate. The reaction is performed at a temperature from about room temperature to about reflux until complete. At reflux temperature, the reactions are typically complete in from about 12 to about 48 hours. The compounds of the invention are then isolated by standard extractive workup and purified by chromatography or crystallization as appropriate. Pharmaceutically acceptable salts are then prepared if necessary or desired under standard conditions.

Compounds of Formula I where R is hydroxy are prepared by reacting a substituted piperidine of Formula II with a glycidyl AR¹ ether of Formula IV under standard nucleophilic displacement conditions. The requisite piperidine and glycidyl ether are combined in a mutual solvent, typically methanol, and the mixture heated to about reflux until the reaction is complete. At reflux temperatures, the reactions are typically complete in from 12 to 48 hours. The compounds of the invention are then isolated by standard extractive workup and purified by chromatography or crystallization as appropriate. Pharmaceutically acceptable salts are then prepared if necessary or desired under standard conditions.

The 3-chloropropyl AR¹ ethers of Formula III are prepared by O-alkylation of an appropriate AR¹ alcohol with 1-bromo-3-chloropropane as illustrated in Synthetic Scheme II, where AR¹ is as previously defined. 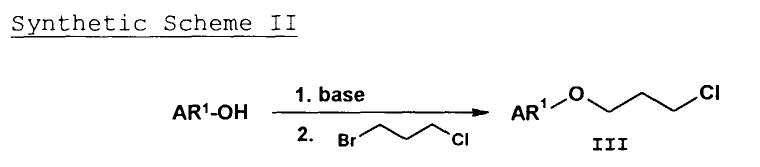 An appropriate alcohol is deprotonated with a suitable base, typically sodium hydride, in a suitable solvent, typically dimethylformamide, at from about 0°C to about room temperature. The resultant anion is then reacted with 1-bromo-3-chloropropane at about room temperature for from about 1 hour to about 2 days. The desired chloropropyl ether is isolated by normal extractive workup. The compound may be used as isolated for subsequent reactions, or purified by chromatography if necessary or desired.

The 3-glycidyl AR¹ ethers of Formula IV are prepared by reaction of an appropriate AR¹ alcohol with a glycidyl-3-nitrobenzenesulfonate as illustrated in Synthetic Scheme III, where AR¹ is as previously defined. 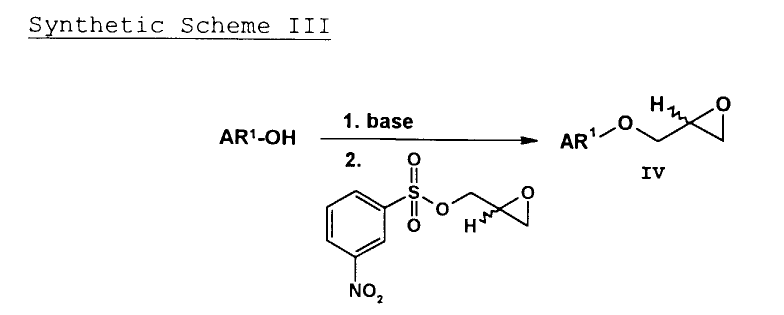 The requisite anion is prepared as described *supra* and is then reacted with with an appropriate glycidyl-3-nitrobenzenesulfonate at room temperature for from about 1 to about 24 hours. The desired glycidyl ether is isolated by normal extractive workup. The compound may be used as isolated for subsequent reactions, or purified by chromatography or crystallization if necessary or desired.

The compounds of Formula II where R' is hydroxy are prepared by the procedure illustrated in Synthetic Scheme IV, where halide is chloro, bromo or iodo and AR² is as previously defined. 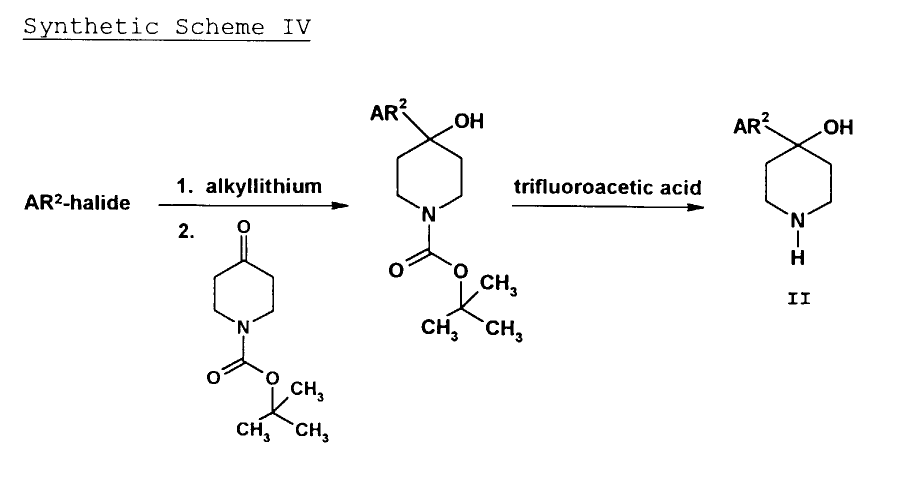 An appropriate AR²-halide is reacted with an alkyllithium, typically n-butyllithium or sec-butyllithium, at about -100 to about -78°C for from 1 to about 4 hours in a suitable solvent, such as diethyl ether or tetrahydro-furan. To the AR²-Li formed in this manner is added 1-tert-butoxycarbonyl-4-piperdone and the reaction is stirred from about 4 to about 24 hours at room temperature. The resultant alcohol is isolated by extractive workup may be used as isolated for subsequent reactions or purified by chromatography if necessary. The alcohol is N-deprotected by reaction with trifluoroacetic acid in a suitable solvent, typically dichloromethane, at room temperature for from about 4 to about 24 hours. Excess acid is neutralized with an appropriate base, typically sodium or potassium hydroxide, and the desired product isolated by normal extractive work up. The 4-hydroxypiperidine may be used as is or purified by chromatography if necessary or desired.

The compounds of Formula II where R' is hydrogen are prepared as illustrated in Synthetic Scheme V where halide and AR² are as previously defined. 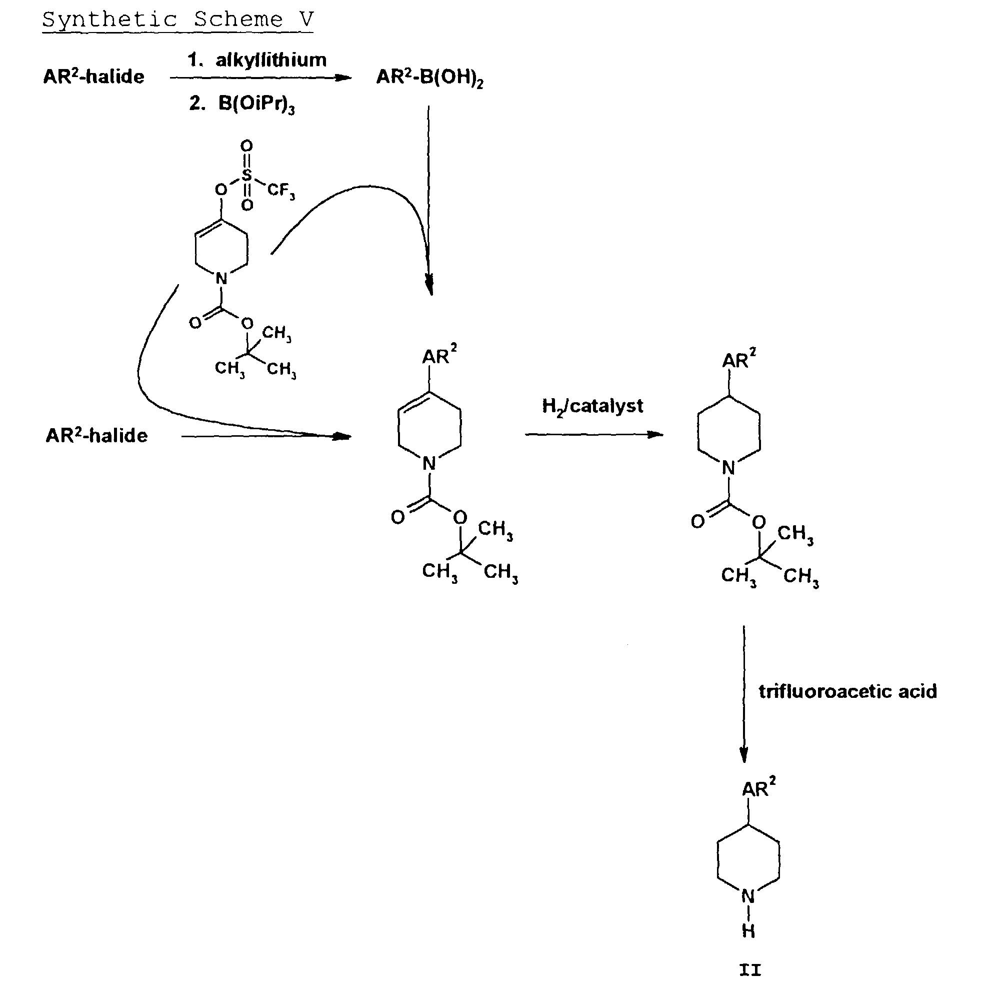 The AR²-halide is reacted with an alkyllithium, typically n-butyllithium or sec-butyllithium, at about -78°C for from 1 to about 4 hours in a suitable solvent, such as diethyl ether or tetrahydrofuran. To the AR²-Li formed in this manner is added triisopropylborate and the reaction is stirred from about 4 to about 24 hours at room temperature. The resultant alcohol is isolated by extractive workup may be used as isolated for subsequent reactions or purified by chromatography or crystallization if necessary. The resultant boronic acid and 1-tert-butoxycarbonyl-4-trifluoromethanesulfonyloxy-1,2,5,6-tetrahydropyridine are reacted together with [1,1-bis(diphenylphosphino)-1-ferrocene]palladium II chloride in tetrahydrofuran containing lithium chloride, aqueous sodium carbonate and methanol. The reaction is performed at about reflux for from about 1 to about 12 hours. The desired tetrahydropyridine is isolated by standard extractive work up and may be used as isolated for subsequent reactions or purified by chromatography if necessary or desired. The 4-substituted-tetrahydropyridine is then hydrogenated in the presence of a precious metal catalyst, typically palladium on carbon, in a suitable solvent, typically a lower alkanol such as methanol or ethanol. The hydrogenation may be performed at about 1 atmosphere at a temperature of from about ambient to reflux. Additional charges of hydrogen may be required to completely reduce the double bond. The piperidine product is isolated by filtration of the reaction mixture and concentration under reduced pressure. The N-tert-butyloxycarbonyl protecting group is removed by treatment with trifluoroacetic acid as previously described to provide the 4-substituted piperidines of Formula II.

Where AR² is quinoxalin-2-yl, the AR²-halide is coupled directly with 1-tert-butoxycarbonyl-4-trifluoromethanesulfonyloxy-1,2,5,6-tetrahydropyridine in the presence of hexamethylditin and [tetrakis(triphenylphosphine)]palladium in 1,4-dioxane containing lithium chloride. The reaction is performed at reflux for about 18 hours. The tetrahydropyridine is isolated and converted to a compound of Formula II by the procedures previously described.

### Preparation I

### 1-tert-butoxycarbonyl-4-piperidone

A solution of 9.0 gm (61.5 mMol) 4-piperidone hydrochloride monohydrate in dioxane/water at 0°C was treated sequentially with aqueous sodium carbonate and 14.4 gm (68 mMol) 2,2-dimethylpropanoic anhydride (BOC anhydride). The resultant slurry was stirred vigorously at room temperature for 18 hours. The reaction mixture was then concentrated under reduced pressure and the residue diluted with ethyl acetate. This mixture was treated with 1.5 M aqueous sodium hydrogen sulfate until the pH was about 2. The layers were separated and the remaining organics were washed with saturated aqueous sodium chloride, dried over sodium sulfate and concentrated under reduced pressure to give 9.8 gm (80%) of the title compound as a tan solid.
EA: Calculated for: C₁₀H₁₇NO₃: Theory: C, 60.28; H, 8.60; N, 7.03. Found: C, 60.12; H, 8.54; N, 7.11.
MS(m/e): 199(M⁺)

The glycidyl aryl ethers required for the synthesis of the compounds of the present invention are prepared from the appropriate alcohol by the procedure described in detail in Preparation II.

### Preparation II

### (2S)-Glycidyl indol-4-yl ether

A suspension of 0.53 gm (13.3 mMol) sodium hydride (60% dispersion in mineral oil) in 25 mL dimethylform-amide was cooled to 0°C under a nitrogen atmosphere. To this suspension were added 1.62 gm (12.2 mMol) 4-hydroxy-indole over 30 minutes and the reaction mixture was allowed to stir at room temperature for 2 hours. To the reaction mixture was then added dropwise a solution of 3.0 gm (11.5 mMol) (2S)-(+)-glycidyl-3-nitrobenzene-sulfonate (Aldrich Chemical Co., Milwaukee, WI, USA) in 10 mL dimethylformamide and the resulting mixture was stirred at room temperature for an additional 1.5 hours. The reaction mixture was then diluted with 100 mL water and extracted well with ethyl acetate. The ethyl acetate phases were combined, washed sequentially with water and saturated aqueous sodium chloride, dried over sodium sulfate and concentrated under reduced pressure. The residual oil was subjected to flash silica gel chromatography, eluting with dichloromethane. Fractions containing product were combined and concentrated under reduced pressure to provide 1.34 gm (62%) of the title compound as a yellow solid.

### Preparation III

### (2S)-Glycidyl 1-methylindol-4-yl ether

A suspension of 0.111 gm (0.28 mMol) sodium hydride (60% dispersion in mineral oil) in dimethylformamide was cooled to 0°C under a nitrogen atmosphere. To this suspension were added 0.50 gm (2.6 mMol) (2S)-glycidyl indol-4-yl ether and the reaction mixture was stirred for 20 minutes at room temperature. To the reaction mixture were then added 0.17 mL (2.8 mMol) iodomethane and the mixture was stirred at room temperature for 18 hours. The reaction mixture was diluted with water and then extracted well with ethyl acetate. The ethyl acetate phases were combined, washed sequentially with water and saturated aqueous sodium chloride, dried over sodium sulfate and concentrated under reduced pressure. The residue was subjected to flash silica gel chromatography, eluting with 20% ethyl acetate in hexane. Fractions containing product were combined and concentrated under reduced pressure to provide 0.376 gm (70%) of the title compound as a white, waxy solid.

The 3-chloropropyl aryl ethers required for the synthesis of the compounds of Formula I are prepared from the appropriate alcohol by the procedure described in detail in Preparation III.

### Preparation IV

### 3-chloropropyl indol-4-yl ether

A suspension of 1.6 gm (39.7 mMol) sodium hydride (60% dispersion in mineral oil) in 80 mL dimethylform-amide was cooled to 0°C under a nitrogen atmosphere. To this suspension were added 5.0 gm (37.6 mMol) 4-hydroxy-indole in portions over 30 minutes. The reaction mixture was stirred at room temperature for 1.5 hours after this addition was complete. To the resulting mixture was then added dropwise a solution of 5.91 gm (37.6 mMol) 1-bromo-3-chloropropane in dimethylformamide and the reaction mixture stirred for 18 hours at room temperature. The reaction mixture was diluted with water and then extracted well with ethyl acetate. The ethyl acetate phases were combined, washed sequentially with water and saturated aqueous sodium chloride, dried over sodium sulfate and concentrated under reduced pressure. The residue was subjected to flash silica gel chromatography, eluting with 10% ethyl acetate in hexane. Fractions containing product were combined and concentrated under reduced pressure to provide 4.62 gm (59%) of the title compound.

The 4-aryl-4-hydroxypiperidines required for the synthesis of the compounds of Formula I are prepared from the appropriate aryl halide and N-protected-4-piperidone by the procedure described in detail in Preparation IV.

### Preparation V

### 4-(isoquinolin-4-yl)-4-hydroxypiperidine

A solution of 6.46 gm (31.1 mMol) 4-bromoisoquinoline in 80 mL tetrahydrofuran was cooled to -100°C under a nitrogen atmosphere. To this solution was added dropwise 28.7 mL (37.3 mMol) sec-butyllithium (1.3 M in hexanes) and the reaction mixture was allowed to stir for 1.5 hours. To the reaction mixture was then added dropwise a solution of 1-tert-butoxycarbonyl-4-piperidone in 20 mL tetrahydrofuran dropwise. The reaction mixture was then stirred for 18 hours at room temperature. The reaction mixture was then partitioned between ethyl acetate and 2N sodium hydroxide. The phases were separated and the aqueous phase extracted well with ethyl acetate. The organic phases were combined, washed saturated aqueous sodium chloride, dried over sodium sulfate and concentrated under reduced pressure. The residue was subjected to silica gel chromatography, eluting with 9:1 dichloromethane:methanol. Fractions containing product were combined and concentrated under reduced pressure to provide 3.95 gm (39%) 1-tert-butoxycarbonyl-4-(isoquin-olin-4-yl)-4-hydroxypiperidine as a yellow solid.

A mixture of 2.0 gm (6.1 mMol) 1-tert-butoxycarbonyl-4-(isoquinolin-4-yl)-4-hydroxypiperidine, and 4 mL trifluoroacetic acid in 20 mL dichloromethane was stirred at room temperature for 18 hours. The reaction mixture was diluted with 2N sodium hydroxide and the phases were separated. The aqueous phase was extracted well with dichloromethane. The organic phases were combined, washed with saturated aqueous sodium chloride, dried over sodium sulfate and concentrated under reduced pressure. The residue was subjected to flash silica gel chromato-graphy, eluting with dichloromethane containing from 10% to 40% methanol and a trace of ammonium hydroxide. Fractions containing product were combined and concentrated under reduced pressure to provide 0.793 gm (57%) of the title compound as a light yellow solid.
MS(m/e): 228(M⁺)
Calculated for C₁₄H₁₆N₂O-0.25 H₂O: Theory: C, 72.94; H, 7.10; N, 12.15. Found: C, 72.97; H, 7.09; N, 12.26.

### Preparation VI

### 4-(quinolin-3-yl)-4-hydroxypiperidine

Beginning with 7.46 gm (35.9 mMol) 3-bromoquinoline, 7.25 gm (62%) N-tert-butoxycarbonyl-4-(quinolin-3-yl)-4-hydroxypiperidine were recovered as a light yellow solid by the procedure described in detail in Preparation IV.

Beginning with 1.5 gm (4.6 mMol) N-tert-butoxycarbonyl-4-(quinolin-3-yl)-4-hydroxypiperidine, 0.645 gm (62%) of the title compound were recovered as a light tan solid by the procedure described in detail in Preparation IV.
MS(m/e): 228(M⁺)
Calculated for C₁₄H₁₆N₂O-0.25 H₂O: Theory: C, 72.23; H, 7.14; N, 12.03. Found: C, 72.41; H, 7.12; N, 12.89.

### Preparation VII

### 1-tert-butoxycarbonyl-4-trifluoromethanesulfonyloxy-1,2,5,6-tetrahydropyridine

A solution of 1.2 mL (8.2 mMol) diisopropylamine in 15 mL tetrahydrofuran was cooled to -78°C. To this solution were added dropwise 5 mL (7.9 mMol) n-butyl-lithium (1.6 M in hexanes) and the reaction mixture was stirred for 1.5 hours at -78°C and was then allowed to warm to room temperature. The resulting solution was cooled again to-78°C and then a solution of 1.56 gm (7.8 mMol) N-tert-butoxycarbonyl-4-piperidone in tetrahydro-furan was added dropwise. After about 30 minutes, a solution of 3.0 gm (8.4 mMol) N-phenyltrifluoromethanesulfonimide in tetrahydrofuran was added dropwise. The reaction mixture was allowed to warm gradually to room temperature and was then concentrated under reduced pressure. The residue was dissolved in dichloromethane and placed on a pad of neutral alumina. The alumina column was eluted with 9:1 hexane:ethyl acetate. Frac-tions containing product were combined and concentrated under reduced pressure to provide 2.24 gm (86%) of the title compound as an oil.

### Preparation VIII

### 4-(isoquinolin-4-yl)piperidine oxalate

A solution of 2.0 gm (9.6 mMol) 4-bromoisoquinoline in 30 mL tetrahydrofuran was cooled to -100°C. To this solution were added dropwise 6.3 mL (10.1 mMol) n-butyllithium (1.6 M in hexane) dropwise, and the resultant solution was stirred for 30 minutes. To this solution was then added dropwise a solution of 4.4 mL (19.2 mMol) triisopropylborate and the reaction mixture was then stirred for 18 hours at room temperature. The reaction mixture was then partitioned between ethyl acetate and saturated aqueous sodium chloride. The phases were separated and the aqueous phase extracted well with ethyl acetate. The combined organic phases were washed with saturated aqueous sodium chloride, dried over sodium sulfate and concentrated under reduced pressure. The residue was sonicated in a mixture of hexane:ethyl acetate. The resulting suspension was filtered to provide 0.57 gm (34%) isoquinolin-4-ylboronic acid as a light orange solid.

A mixture of 2.5 gm (14.5 mMol) isoquinolin-4-ylboronic acid, 3.43 gm (10.3 mMol) 1-tert-butoxycarbonyl-4-trifluoromethanesulfonyloxy-1,2,5,6-tetrahydropyridine, 1.3 gm (30.9 mMol) lithium chloride, 0.04 gm (0.05 mMol) [1,1'-bis(diphenylphosphino)-1-ferrocene]palladium II chloride, and 2 mL 2M aqueous sodium carbonate in about 20 mL tetrahydrofuran containing a few drops of methanol was stirred at reflux for about 4 hours. The reaction was cooled to room temperature and then partitioned between ethyl acetate and 2N sodium hydroxide. The phases were separated and the aqueous phase was extracted well with ethyl acetate. The organic phases were combined, washed with saturated aqueous sodium chloride, dried over sodium sulfate and concentrated under reduced pressure. The residue was subjected to flash silica gel chromatography, eluting with 3:2 hexane:ethyl acetate. Fractions containing product were combined and concentrated under reduced pressure to provide 0.216 gm (57%) 1-tert-butoxycarbonyl-4-(isoquinolin-4-yl)-1,2,5,6-tetrahydro-pyridine as a light yellow oil.

A mixture of 0.91 gm (2.9 mMol) 1-tert-butoxycarbonyl-4-(isoquinolin-4-yl)-1,2,5,6-tetrahydropyridine and 0.1 gm 5% palladium on carbon in 40 mL methanol was stirred at room temperature for 3 days under a hydrogen atmosphere. The reaction mixture was then filtered and the filtrate concentrated under reduced pressure. The residue was subjected to flash silica gel chromatography, eluting with 1:1 ethyl acetate:hexane. Fractions containing product were combined and concentrated under reduced pressure to provide 0.57 gm (63%) of 1-tert-butoxycarbonyl-4-(isoquinolin-4-yl)piperidine as a clear oil.

A mixture of 0.57 gm (1.8 mMol) 1-tert-butoxycarbonyl-4-(isoquinolin-4-yl)piperidine and 6 mL trifluoroacetic acid in 6 mL dichloromethane was stirred at room temperature for 18 hours. The reaction mixture was diluted with 2N sodium hydroxide and the phases were separated. The aqueous phase was extracted well with dichloromethane. The organic phases were combined, washed with saturated aqueous sodium chloride, dried over sodium sulfate and concentrated under reduced pressure. The residue was subjected to flash silica gel chromato-graphy, eluting with dichloromethane containing from 10% to 40% methanol and a trace of ammonium hydroxide. Fractions containing product were combined and concentrated under reduced pressure to provide 0.242 gm (63%) of 4-(isoquinolin-4-yl)piperidine as a tan solid. A portion of this material was converted to the oxalate salt to provide the title compound.
MS(m/e): 212(M⁺)
Calculated for C₁₆H₁₈N₂O₄-0.25 H₂O: Theory: C, 62.63; H, 6.08; N, 9.13. Found: C, 62.22; H, 5.99; N, 9.04.

### Preparation IX

### 4-(quinolin-3-yl)piperidine

Beginning with 3-bromoquinoline, the title compound was recovered as a clear oil by the procedure described in detail in Preparation VII.
MS(m/e): 212(M⁺)

### Preparation X

### 4-(pyridin-3-yl)piperidine

Beginning with 3-bromopyridine, the title compound was recovered as a light tan waxy solid by the procedure described in detail in Preparation VII. A small amount of this material was converted to the oxalate salt.
MS(m/e): 162(M⁺)
Calculated for C₁₂H₁₆N₂O₄-0.75 H₂O: Theory: C, 54.23; H, 6.07; N, 10.53. Found: C, 54.30; H, 5.96; N, 10.14.

### Preparation XI

### 4-(quinoxalin-2-yl)piperidine

A mixture of 1.4 gm (8.5 mMol) 2-chloroquinoxaline, 2.82 gm (8.5 mMol) 1-tert-butoxycarbonyl-4-trifluoromethanesulfonyloxy-1,2,5,6-tetrahydropyridine, 2.8 gm (8.5 mMol) hexamethylditin, 1.08 gm (25.5 mMol) lithium chloride, and 0.491 gm (0.43 mMol) [tetrakis(triphenylphosphine)]palladium in dioxane was stirred at reflux for 18 hours. The reaction mixture was cooled to room temperature and then poured into a mixture of saturated aqueous potassium fluoride and ethyl acetate. After stirring for two hours, the phases were separated. The organic phase was washed with saturated aqueous sodium chloride, dried over magnesium sulfate and concentrated under reduced pressure. The residue was subjected to flash silica gel chromatography, eluting with a gradient of 100:0 to 25:3 hexane:ethyl acetate. Fractions con-taining product were combined and concentrated under reduced pressure to provide 1.43 gm (54%) 1-tert-butoxycarbonyl-4-(quinoxalin-2-yl)-1,2,5,6-tetrahydropyridine as a light yellow oil.

A mixture of 0.55 gm (1.8 mMol) 1-tert-butoxycarbon-yl-4-(quinoxalin-2-yl)-1,2,5,6-tetrahydropyridine and 0.1 gm 5% palladium on carbon in 10 mL methanol was stirred at room temperature for 45 minutes under a hydrogen atmosphere. The reaction mixture was then filtered and the filtrate concentrated under reduced pressure to provide 0.47 gm (84%) of 1-tert-butoxycarbonyl-4-(quinoxalin-2-yl)piperidine as a yellow oil.

A mixture of 0.47 gm (1.5 mMol) 1-tert-butoxycarbonyl-4-(quinoxalin-2-yl)piperidine and 5 mL trifluoroacetic acid in 5 mL dichloromethane was stirred at room temperature for 18 hours. The reaction mixture was diluted with 2N sodium hydroxide and the phases were separated. The aqueous phase was extracted well with dichloromethane. The organic phases were combined, washed with saturated aqueous sodium chloride, dried over sodium sulfate and concentrated under reduced pressure. The residue was subjected to flash silica gel chromatography, eluting with dichloromethane containing from 10% to 40% methanol and a trace of ammonium hydroxide. Fractions containing product were combined and concentrated under reduced pressure to provide 0.133 gm (42%) of the title compound as a tan foam.

### EXAMPLE 1

### 1-(3-phenoxyprop-1-yl)-4-hydroxy-4-(quinolin-3-yl)piperidine oxalate

A mixture of 0.112 gm (0.7 mMol) 3-chloropropyl phenyl ether, 0.150 gm (0.7 mMol) 4-hydroxy-4-(quinolin-3-yl)piperidine, and 0.136 gm (1.5 mMol) potassium carbonate in 5 mL acetonitrile was heated to reflux for 18 hours. The reaction mixture was then cooled to room temperature and partitioned between ethyl acetate and 2N sodium hydroxide. The phases were separated and the aqueous phase extracted well with ethyl acetate. The combined organic extracts were washed with saturated aqueous sodium chloride, dried over sodium sulfate and concentrated under reduced pressure. The residue was subjected to flash silica gel chromatography, eluting with 25:2 dichloromethane:methanol. Fractions containing product were combined and concentrated under reduced pressure to provide 0.142 gm (60%) of 1-(3-phenoxyprop-1-yl)-4-hydroxy-4-(quinolin-3-yl)piperidine as a light yellow, viscous oil. The oil was converted to the oxalate salt to provide the title compound.
m.p. = 76°C
MS(m/e): 362(M⁺)

The compounds of Examples 2-12 were prepared by the procedure described in detail in Example 1.

### EXAMPLE 2

### 1-(3-(2-tert-butylphenoxy)prop-1-yl)-4-hydroxy-4-(quinolin-3-yl)piperidine

Beginning with 0.298 gm (1.3 mMol) 3-chloropropyl 2-tert-butylphenyl ether and 0.300 gm (1.3 mMol) 4-hydroxy-4-(quinolin-3-yl)piperidine, 0.212 gm (39%) of the title compound were recovered as a white, waxy solid.
MS(m/e): 418(M⁺)
Calculated for C₂₇H₃₄N₂O₂: Theory: C, 77.48; H, 8.19; N, 6.69. Found: C, 77.64; H, 8.40; N, 6.91.

### EXAMPLE 3

### 1-(3-(3,4-dimethylphenoxy)prop-1-yl)-4-hydroxy-4-(quinolin-3-yl)piperidine

Beginning with 0.218 gm (1.1 mMol) 3-chloropropyl 3,4-dimethylphenyl ether and 0.250 gm (1.1 mMol) 4-hydroxy-4-(quinolin-3-yl)piperidine, 0.114 gm (27%) of the title compound were recovered as a white solid.
MS(m/e): 391(M+1)
Calculated for C₂₅H₃₀N₂O₂-0.25 H₂O: Theory: C, 76.01; H, 7.85; N, 7.15. Found: C, 75.96; H, 7.57; N, 7.07.

### EXAMPLE 4

### 1-(3-(2,4-dimethylphenoxy)prop-1-yl)-4-hydroxy-4-(quinolin-3-yl)piperidine

Beginning with 0.235 gm (1.2 mMol) 3-chloropropyl 2,4-dimethylphenyl ether and 0.270 gm (1.2 mMol) 4-hydroxy-4-(quinolin-3-yl)piperidine, 0.209 gm (45%) of the title compound were recovered as a white solid.
MS(m/e): 390(M⁺)
Calculated for C₂₅H₃₀N₂O₂-0.25 H₂O: Theory: C, 76.01; H, 7.85; N, 7.15. Found: C, 76.08; H, 7.56; N, 7.07.

### EXAMPLE 5

### 1-(3-(2,4-dichlorophenoxy)prop-1-yl)-4-hydroxy-4-(quinolin-3-yl)piperidine oxalate

Beginning with 0.314 gm (1.3 mMol) 3-chloropropyl 2,4-dichlorophenyl ether and 0.300 gm (1.3 mMol) 4-hydroxy-4-(quinolin-3-yl)piperidine, 0.277 gm (49%) of 1-(3-(2,4-dichlorophenoxy)prop-1-yl)-4-hydroxy-4-(quinolin-3-yl)piperidine were recovered as a white, waxy solid. A portion was converted to the oxalate salt.
m.p. = 118°C
MS(m/e): 430(M⁺)
Calculated for C₂₃H₂₄N₂O₂Cl₂-C₂H₂O₄: Theory: C, 57.59; H, 5.02; N, 5.37. Found: C, 57.83; H, 5.07; N, 5.49.

### EXAMPLE 6

### 1-(3-(2,6-dimethoxyphenoxy)prop-1-yl)-4-hydroxy-4-(quinolin-3-yl)piperidine oxalate

Beginning with 0.303 gm (1.3 mMol) 3-chloropropyl 2,4-dichlorophenyl ether and 0.300 gm (1.3 mMol) 4-hydroxy-4-(quinolin-3-yl)piperidine, 0.318 gm (57%) of 1-(3-(2,6-dimethoxyphenoxy)prop-1-yl)-4-hydroxy-4-(quinolin-3-yl)piperidine were recovered as a white foam. A portion was converted to the oxalate salt.
m.p. = 102°C
MS(m/e): 422(M⁺)

### EXAMPLE 7

### 1-(3-(indol-4-yloxy)prop-1-yl)-4-hydroxy-4-(quinolin-3-yl)piperidine

Beginning with 0.253 gm (1.2 mMol) 3-chloropropyl indol-4-yl ether and 0.275 gm (1.2 mMol) 4-hydroxy-4-(quinolin-3-yl)piperidine, 0.253 gm (52%) of the title compound were recovered as a white solid.
MS(m/e): 401(M⁺)
Calculated for C₂₅H₂₇N₃O₂: Theory: C, 74.79; H, 6.78; N, 10.47. Found: C, 74.66; H, 6.87; N, 10.43.

### EXAMPLE 8

### 1-(3-(indol-4-yloxy)prop-1-yl)-4-hydroxy-4-(isoquinolin-4-yl)piperidine

Beginning with 0.184 gm (0.88 mMol) 3-chloropropyl indol-4-yl ether and 0.200 gm (0.88 mMol) 4-hydroxy-4-(isoquinolin-4-yl)piperidine, 0.169 gm (48%) of the title compound were recovered as a light yellow solid.
MS(m/e): 401(M⁺)
Calculated for C₂₅H₂₇N₃O₂: Theory: C, 74.79; H, 6.78; N, 10.47. Found: C, 74.51; H, 6.76; N, 10.34.

### EXAMPLE 9

### 1-(3-(indol-5-yloxy)prop-1-yl)-4-hydroxy-4-(quinolin-3-yl)piperidine

Beginning with 0.275 gm (1.3 mMol) 3-chloropropyl indol-5-yl ether and 0.300 gm (1.3 mMol) 4-hydroxy-4-(quinolin-3-yl)piperidine, 0.238 gm (45%) of the title compound were recovered as an off-white solid.
MS(m/e): 401(M⁺)
Calculated for C₂₅H₂₇N₃O₂: Theory: C, 74.79; H, 6.78; N, 10.47. Found: C, 74.77; H, 6.73; N, 10.36.

### EXAMPLE 10

### 1-(3-(2,2-dimethyl-2,3-dihydrobenzofur-7-yloxy)prop-1-yl)-4-hydroxy-4-(quinolin-3-yl)piperidine

Beginning with 0.316 gm (1.3 mMol) 3-chloropropyl 2,2-dimethyl-2,3-dihydrobenzofur-7-yl ether and 0.300 gm (1.3 mMol) 4-hydroxy-4-(quinolin-3-yl)piperidine, 0.262 gm (46%) of the title compound were recovered as a white solid. MS(m/e): 432(M⁺)
Calculated for C₂₇H₃₂N₂O₃: Theory: C, 74.97; H, 7.46; N, 6.48. Found: C, 75.25; H, 7.73; N, 6.68.

### EXAMPLE 11

### 1-(3-(indol-4-yloxy)prop-l-yl)-4-(isoquinolin-4-yl)piperidine oxalate

Beginning with 0.109 gm (0.5 mMol) 3-chloropropyl indol-4-yl ether and 0.110 gm (0.5 mMol) 4-(isoquinolin-4-yl)piperidine, 0.112 gm (56%) of 1-(3-(indol-4-yloxy)prop-1-yl)-4-(isoquinolin-4-yl)piperidine were recovered as an off-white solid. A portion was converted to the oxalate salt. m.p. = 99"C
MS(m/e): 385(M⁺)
Calculated for C₂₅H₂₇N₃O-C₂H₂O₄-0.75 H₂O: Theory: C, 66.32; H, 6.24; N, 8.59. Found: C, 66.54; H, 6.23; N, 8.88.

### EXAMPLE 12

### 1-(3-(indol-4-yloxy)prop-1-yl)-4-(quinolin-3-yl)piperidine

Beginning with 0.175 gm (0.83 mMol) 3-chloropropyl indol-4-yl ether and 0.177 gm (0.83 mMol) 4-(quinolin-3-yl)piperidine, 0.217 gm (68%) of the title compound were recovered as an off-white solid.
MS(m/e): 385(M⁺)
Calculated for C₂₅H₂₇N₃O: Theory: C, 77.89; H, 7.06; N, 10.90. Found: C, 77.66; H, 7.07; N, 10.68.

### EXAMPLE 13

### 1-((2R,S)-hydroxy-3-phenoxyprop-1-yl)-4-hydroxy-4-(quinolin-3-yl)piperidine

A mixture of 0.18 mL (1.3 mMol) (2R,S)-glycidyl phenyl ether and 0.300 gm (1.3 mMol) 4-hydroxy-4-(quinolin-3-yl)piperidine in 10 mL methanol was heated at reflux for 18 hours. The reaction mixture was then cooled to room temperature and partitioned between ethyl acetate and 2N sodium hydroxide. The phases were separated and the aqueous phase extracted well with ethyl acetate. The combined organic phases were washed with saturated aqueous sodium chloride, dried over sodium sulfate and concentrated under reduced pressure. The residue was subjected to flash silica gel chromatography, eluting with 25:1 dichloromethane:methanol. Fractions containing product were combined and concentrated under reduced pressure to provide 0.209 gm (42%) of the title compound as a tan, waxy solid.
MS(m/e): 378(M⁺)
Calculated for C₂₃H₂₆N₂O₃: Theory: C, 72.99; H, 6.92; N, 7.40. Found: C, 72.53; H, 6.78; N, 7.33.

The compounds of Examples 14-60 were prepared by the procedure described in detail in Example 13.

### EXAMPLE 14

### 1-((2S)-hydroxy-3-(4-methylphenoxy)prop-1-yl)-4-hydroxy-4-(quinolin-3-yl)piperidine

Beginning with 0.108 gm (0.7 mMol) (S)-glycidyl 4-methylphenyl ether and 0.150 gm (0.7 mMol) 4-hydroxy-4-(quinolin-3-yl)piperidine, 0.175 gm (68%) of the title compound were recovered as a white solid.
MS(m/e): 392(M⁺)
Calculated for C₂₄H₂₈N₂O₃-0.5 H₂O: Theory: C, 71.80; H, 7.28; N, 6.98. Found: C, 72.01; H, 6.99; N, 7.03.

### EXAMPLE 15

### 1-((2S)-hydroxy-3-(4-ethylphenoxy)prop-1-yl)-4-hydroxy-4-(quinolin-3-yl)piperidine

Beginning with 0.117 gm (0.7 mMol) (S)-glycidyl 4-ethylphenyl ether and 0.150 gm (0.7 mMol) 4-hydroxy-4-(quinolin-3-yl)piperidine, 0.100 gm (37%) of the title compound were recovered as a waxy solid. This material was converted to the oxalate salt.
m.p. = 76°C
MS(m/e): 406(M⁺)
Calculated for C₂₅H₃₀N₂O₃-C₂H₂O₄: Theory: C, 65.31; H, 6.50; N, 5.64. Found: C, 65.23; H, 6.46; N, 5.56.

### EXAMPLE 16

### 1-((2S)-hydroxy-3-(4-isopropylphenoxy)prop-1-yl)-4-hydroxy-4-(quinolin-3-yl)piperidine

Beginning with 0.126 gm (0.7 mMol) (S)-glycidyl 4-isopropylphenyl ether and 0.150 gm (0.7 mMol) 4-hydroxy-4-(quinolin-3-yl)piperidine, 0.121 gm (44%) of the title compound were recovered as a light yellow solid. This material was converted to the oxalate salt.
MS(m/e): 421(M+1)
Calculated for C₂₅H₃₀N₂O₃-C₂H₂O₄-0.5 H₂O: Theory: C, 64.72; H, 6.79; N, 5.39. Found: C, 64.61; H, 6.75; N, 5.39.

### EXAMPLE 17

### 1-((2S)-hydroxy-3-(4-tert-butylphenoxy)prop-1-yl)-4-hydroxy-4-(quinolin-3-yl)piperidine

Beginning with 0.136 gm (0.7 mMol) (S)-glycidyl 4-tert-butylphenyl ether and 0.150 gm (0.7 mMol) 4-hydroxy-4-(quinolin-3-yl)piperidine, 0.115 gm (40%) of the title compound were recovered as a white foam.
MS(m/e): 434(M⁺)
Calculated for C₂₇H₃₄N₂O₃-0.5 H₂O: Theory: C, 73.11; H, 7.95; N, 6.32. Found: C, 72.92; H, 7.53; N, 6.48.

### EXAMPLE 18

### 1-((2S)-hydroxy-3-(4-chlorophenoxy)prop-1-yl)-4-hydroxy-4-(quinolin-3-yl)piperidine

Beginning with 0.121 gm (0.7 mMol) (S)-glycidyl 4-chlorophenyl ether and 0.150 gm (0.7 mMol) 4-hydroxy-4-(quinolin-3-yl)piperidine, 0.156 gm (58%) of the title compound were recovered as an off-white foam.
Calculated for C₂₃H₂₅N₂O₃Cl: Theory: C, 66.90; H, 6.10; N, 6.78. Found: C, 66.73; H, 6.00; N, 6.94.
This foam was converted to the oxalate salt.
m.p. = 81°C
MS(m/e): 413(M⁺)
Calculated for C₂₃H₂₅N₂O₃Cl-C₂H₂O₄: Theory: C, 59.70; H, 5.41; N, 5.57. Found: C, 59.58; H, 5.70; N, 5.28.

### EXAMPLE 19

### 1-((2S)-hydroxy-3-(4-iodophenoxy)prop-1-yl)-4-hydroxy-4-(quinolin-3-yl)piperidine

Beginning with 0.181 gm (0.7 mMol) (S)-glycidyl 4-iodophenyl ether and 0.150 gm (0.7 mMol) 4-hydroxy-4-(quinolin-3-yl)piperidine, 0.201 gm (61%) of the title compound were recovered as a white solid. This solid was converted to the oxalate salt.
MS(m/e): 504(M⁺)
Calculated for C₂₃H₂₅N₂O₃I-C₂H₂O₄: Theory: C, 50.52; H, 4.58; N, 4.72. Found: C, 50.40; H, 4.70; N, 4.42.

### EXAMPLE 20

### 1-((2S)-hydroxy-3-(4-trifluoromethylphenoxy)prop-1-yl)-4-hydroxy-4-(quinolin-3-yl)piperidine

Beginning with 0.143 gm (0.7 mMol) (S)-glycidyl 4-trifluoromethylphenyl ether and 0.150 gm (0.7 mMol) 4-hydroxy-4-(quinolin-3-yl)piperidine, 0.200 gm (68%) of the title compound were recovered as a white solid.
MS(m/e): 446(M⁺)
Calculated for C₂₄H₂₅N₂O₃F₃-0.25 H₂O: Theory: C, 63.92; H, 5.70; N, 6.21. Found: C, 63.85; H, 5.58; N, 6.22.

### EXAMPLE 21

### 1-((2S)-hydroxy-3-(2-methoxyphenoxy)prop-1-yl)-4-hydroxy-4-(quinolin-3-yl)piperidine

Beginning with 0.118 gm (0.7 mMol) (S)-glycidyl 2-methoxyphenyl ether and 0.150 gm (0.7 mMol) 4-hydroxy-4-(quinolin-3-yl)piperidine, 0.145 gm (54%) of the title compound were recovered as a white foam.
Calculated for C₂₄H₂₈N₂O₄-0.75 H₂O: Theory: C, 68.31; H, 7.05; N, 6.64. Found: C, 68.61; H, 6.83; N, 6.56.
[a]_{D}²⁵(methanol) = +11.719°
This foam was converted to the oxalate salt.
m.p. = 182-185°C
MS(m/e): 408(M⁺)
Calculated for C₂₄H₂₈N₂O₄-C₂H₂O₄: Theory: C, 62.64; H, 6.07; N, 5.62. Found: C, 62.51; H, 6.13; N, 5.46.
[a]_{D}²⁵(methanol) = -8.741°

### EXAMPLE 22

### 1-((2S)-hydroxy-3-(3-methoxyphenoxy)prop-1-yl)-4-hydroxy-4-(quinolin-3-yl)piperidine

Beginning with 0.118 gm (0.7 mMol) (S)-glycidyl 3-methoxyphenyl ether and 0.150 gm (0.7 mMol) 4-hydroxy-4-(quinolin-3-yl)piperidine, 0.179 gm (67%) of the title compound were recovered as a white foam.
Calculated for C₂₄H₂₈N₂O₄-0.25 H₂O: Theory: C, 69.80; H, 6.96; N, 6.78. Found: C, 69.57; H, 6.74; N, 6.92.
[a]_{D}²⁵(methanol) = +5.254°
This foam was converted to the oxalate salt.
m.p. = 79°C
MS(m/e): 408(M⁺)
Calculated for C₂₄H₂₈N₂O₄-C₂H₂O₄-2H₂O Theory: C, 58.42; H, 6.41; N, 5.24. Found: C, 58.45; H, 5.67; N, 5.27.
[a]_{D}²⁵(methanol) = -15.444°

### EXAMPLE 23

### 1-((2S)-hydroxy-3-(4-methoxyphenoxy)prop-1-yl)-4-hydroxy-4-(quinolin-3-yl)piperidine

Beginning with 0.118 gm (0.7 mMol) (S)-glycidyl 4-methoxyphenyl ether and 0.150 gm (0.7 mMol) 4-hydroxy-4-(quinolin-3-yl)piperidine, 0.168 gm (63%) of the title compound were recovered as a white waxy solid.
Calculated for C₂₄H₂₈N₂O₄: Theory: C, 70.57; H, 6.91; N, 6.86. Found: C, 70.58; H, 7.04; N, 7.01.
[a]_{D}²⁵(methanol) = +12.635°
This foam was converted to the oxalate salt.
m.p. = 63°C
MS(m/e): 408(M⁺)
Calculated for C₂₄H₂₈N₂O₄-C₂H₂O₄-0.75 H₂O: Theory: C, 60.99; H, 6.20; N, 5.47. Found: C, 60.89; H, 6.34; N, 5.47.
[a]_{D}²⁵(methanol) = -7.843°

### EXAMPLE 24

### 1-((2S)-hydroxy-3-(4-trifluoromethoxyphenoxy)prop-l-yl)-4-hydroxy-4-(quinolin-3-yl)piperidine

Beginning with 0.154 gm (0.7 mMol) (S)-glycidyl 4-trifluoromethoxyphenyl ether and 0.150 gm (0.7 mMol) 4-hydroxy-4-(quinolin-3-yl)piperidine, 0.207 gm (68%) of the title compound were recovered as a white foam. This foam was converted to the oxalate salt.
MS(m/e): 462(M⁺)
Calculated for C₂₄H₂₈N₂O₄F₃: Theory: C, 56.52; H, 4.93; N, 5.07. Found: C, 56.46; H, 4.77; N, 4.86.

### EXAMPLE 25

### 1-((2S)-hydroxy-3-(4-phenoxyphenoxy)prop-1-yl)-4-hydroxy-4-(quinolin-3-yl)piperidine

Beginning with 0.169 gm (0.7 mMol) (S)-glycidyl 4-phenoxyphenyl ether and 0.150 gm (0.7 mMol) 4-hydroxy-4-(quinolin-3-yl)piperidine, 0.117 gm (38%) of the title compound were recovered as a light yellow foam. This foam was converted to the oxalate salt.
m.p. = 63°C
MS(m/e): 470(M⁺)
Calculated for C₂₉H₃₀N₂O₄-C₂H₂O₄: Theory: C, 66.42; H, 5.75; N, 5.00. Found: C, 66.23; H, 5.88; N, 5.07.

### EXAMPLE 26

### 1-((2S)-hydroxy-3-(2-methylthiophenoxy)prop-1-yl)-4-hydroxy-4-(quinolin-3-yl)piperidine

Beginning with 0.300 gm (1.5 mMol) (S)-glycidyl 2-methylthiophenyl ether and 0.349 gm (1.5 mMol) 4-hydroxy-4-(quinolin-3-yl)piperidine, 0.333 gm (51%) of the title compound were recovered as a white solid.
Calculated for C₂₄H₂₈N₂O₃S: Theory: C, 67.90; H, 6.65; N, 6.60. Found: C, 68.08; H, 6.70; N, 6.63.
[a]_{D}²⁵(methanol) = -3.984°
This foam was converted to the oxalate salt.
m.p. = 186-189°C
MS(m/e): 424(M⁺)
Calculated for C₂₄H₂₈N₂O₃S-C₂H₂O₄: Theory: C, 60.69; H, 5.88; N, 5.44. Found: C, 60.69; H, 5.74; N, 5.43.
[a]_{D}²⁵(methanol) = -30,361°

### EXAMPLE 27

### 1-((2S)-hydroxy-3-(4-methylthiophenoxy)prop-1-yl)-4-hydroxy-4-(quinolin-3-yl)piperidine

Beginning with 0.169 gm (0.7 mMol) (S)-glycidyl 4-methylthiophenyl ether and 0.150 gm (0.7 mMol) 4-hydroxy-4-(quinolin-3-yl)piperidine, 0.104 gm (22%) of the title compound were recovered as a white solid. This solid was converted to the oxalate salt.
MS(m/e): 424(M⁺)
Calculated for C₂₄H₂₈N₂O₃S-C₂H₂O₄: Theory: C, 60.69; H, 5.88; N, 5.44. Found: C, 60.95; H, 6.07; N, 5.53.

### EXAMPLE 28

### 1-((2S)-hydroxy-3-(2,3-dimethylphenoxy)prop-1-yl)-4-hydroxy-4-(quinolin-3-yl)piperidine

Beginning with 0.117 gm (0.7 mMol) (S)-glycidyl 2,3-dimethylphenyl ether and 0.150 gm (0.7 mMol) 4-hydroxy-4-(quinolin-3-yl)piperidine, 0.152 gm (57%) of the title compound were recovered as an off-white solid.
Calculated for C₂₅H₃₀N₂O₃: Theory: C, 73.86; H, 7.44; N, 6.89. Found: C, 73.59; H, 7.26; N, 6.90.
[a]_{D}²⁵(methanol) = +5.714°
This solid was converted to the oxalate salt.
m.p. = 72°C
MS(m/e): 406(M⁺)
Calculated for C₂₅H₃₀N₂O₃-C₂H₂O₄: Theory: C, 65.31; H, 6.50; N, 5.64. Found: C, 65.13; H, 6.58; N, 5.63.
[a]_{D}²⁵(methanol) = -21.016°

### EXAMPLE 29

### 1-((2S)-hydroxy-3-(3,4-dimethylphenoxy)prop-1-yl)-4-hydroxy-4-(quinolin-3-yl)piperidine

Beginning with 0.117 gm (0.7 mMol) (S)-glycidyl 3,4-dimethylphenyl ether and 0.150 gm (0.7 mMol) 4-hydroxy-4-(quinolin-3-yl)piperidine, 0.163 gm (61%) of the title compound were recovered as an off-white solid.
Calculated for C₂₅H₃₀N₂O₃: Theory: C, 73.86; H, 7.44; N, 6.89. Found: C, 73.64; H, 7.53; N, 6.82.
[a]_{D}²⁵(methanol) = +10.0381°
This solid was converted to the oxalate salt.
m.p. = 117°C
MS(m/e): 406(M⁺)
[a]_{D}²⁵(methanol) = -5.455°

### EXAMPLE 30

### 1-((2S)-hydroxy-3-(2,4-dimethylphenoxy)prop-1-yl)-4-hydroxy-4-(quinolin-3-yl)piperidine

Beginning with 0.180 gm (1.0 mMol) (S)-glycidyl 2,4-dimethylphenyl ether and 0.231 gm (1.0 mMol) 4-hydroxy-4-(quinolin-3-yl)piperidine, 0.318 gm (77%) of the title compound were recovered as a light pink solid.
Calculated for C₂₅H₃₀N₂O₃: Theory: C, 73.86; H, 7.44; N, 6.89. Found: C, 73.70; H, 7.28; N, 6.61.
[a]_{D}²⁵(methanol) = +7.38°
This solid was converted to the oxalate salt.
m.p. = 102°C
MS(m/e): 406(M⁺)
[a]_{D}²⁵(methanol) = -5.747°

### EXAMPLE 31

### 1-((2S)-hydroxy-3-(2,4-dichlorophenoxy)prop-1-yl)-4-hydroxy-4-(quinolin-3-yl)piperidine

Beginning with 0.144 gm (0.7 mMol) (S)-glycidyl 2,4-dichlorophenyl ether and 0.150 gm (0.7 mMol) 4-hydroxy-4-(quinolin-3-yl)piperidine, 0.176 gm (60%) of the title compound were recovered as an off-white solid.
Calculated for C₂₃H₂₄N₂O₃Cl₂: Theory: C, 61.75; H, 5.41; N, 6.26. Found: C, 61.50; H, 5.60; N, 6.13.
[a]_{D}²⁵(methanol) = +8.913°
This solid was converted to the oxalate salt.
m.p. = 86°C
MS(m/e): 447(M⁺)
[a]_{D}²⁵(methanol) = -3.521°

### EXAMPLE 32

### 1-((2S)-hydroxy-3-(2-methoxy-4-methylphenoxy)prop-1-yl)-4-hydroxy-4-(quinolin-3-yl)piperidine

Beginning with 0.091 gm (0.7 mMol) (S)-glycidyl 2-methoxy-4-methylphenyl ether and 0.150 gm (0.7 mMol) 4-hydroxy-4-(quinolin-3-yl)piperidine, 0.142 gm (59%) of the title compound were recovered as an off-white foam.
Calculated for C₂₅H₃₀N₂O₄-0.50 H₂O: Theory: C, 69.58; H, 7.24; N, 6.49. Found: C, 69.45; H, 7.24; N, 6.43.
This foam was converted to the oxalate salt.
m.p. = 81°C
MS(m/e): 422(M⁺)

### EXAMPLE 33

### 1-((25)-hydroxy-3-(2-methyl-4-methylthiophenoxy)prop-1-yl)-4-hydroxy-4-(quinolin-3-yl)piperidine

Beginning with 0.138 gm (0.7 mMol) (S)-glycidyl 2-methyl-4-methylthiophenyl ether and 0.150 gm (0.7 mMol) 4-hydroxy-4-(quinolin-3-yl)piperidine, 0.184 gm (64%) of the title compound were recovered as a light yellow foam.
Calculated for C₂₅H₃₀N₂O₃S: Theory: C, 68.46; H, 6.90; N, 6.39. Found: C, 68.19; H, 6.67; N, 6.33.
This foam was converted to the oxalate salt.
m.p. = 81°C
MS(m/e): 438(M⁺)
Calculated for C₂₅H₃₀N₂O₃S-C₂H₂O₄: Theory: C, 61.35; H, 6.10; N, 5.30. Found: C, 61.55; H, 6.14; N, 5.18.

### EXAMPLE 34

### 1-((2S)-hydroxy-3-(3-methyl-4-acetylphenoxy)prop-1-yl)-4-hydroxy-4-(quinolin-3-yl)piperidine

Beginning with 0.135 gm (0.7 mMol) (S)-glycidyl 3-methyl-4-acetylphenyl ether and 0.150 gm (0.7 mMol) 4-hydroxy-4-(quinolin-3-yl)piperidine, 0.147 gm (52%) of the title compound were recovered as an off-white foam. This foam was converted to the oxalate salt.
MS(m/e): 435(M+1)
Calculated for C₂₆H₃₀N₂O₄-C₂H₂O₄-0.5 H₂O Theory: C, 63.03; H, 6.23; N, 5.25. Found: C, 62.87; H, 6.02; N, 5.31.

### EXAMPLE 35

### 1-((2S)-hydroxy-3-(2-methyl-4-acetylphenoxy)prop-1-yl)-4-hydroxy-4-(quinolin-3-yl)piperidine

Beginning with 0.135 gm (0.7 mMol) (S)-glycidyl 2-methyl-4-acetylphenyl ether and 0.150 gm (0.7 mMol) 4-hydroxy-4-(quinolin-3-yl)piperidine, 0.134 gm (49%) of the title compound were recovered as an off-white foam.
Calculated for C₂₆H₃₀N₂O₄ Theory: C, 71.87; H, 6.96; N, 6.45. Found: C, 71.71; H, 6.86; N, 6.42.
This foam was converted to the oxalate salt.
m.p. = 90°C
MS(m/e): 435(M+1)

### EXAMPLE 36

### 1-((2S)-hydroxy-3-(2-benzoyl-4-methylphenoxy)prop-1-yl)-4-hydroxy-4-(quinolin-3-yl)piperidine

Beginning with 0.176 gm (0.7 mMol) (S)-glycidyl 2-benzoyl-4-methylphenyl ether and 0.150 gm (0.7 mMol) 4-hydroxy-4-(quinolin-3-yl)piperidine, 0.199 gm (61%) of the title compound were recovered as an off-white foam.
Calculated for C₃₁H₃₂N₂O₄: Theory: C, 74.98; H, 6.49; N, 5.64. Found: C, 74.82; H, 6.26; N, 5.58.
This foam was converted to the oxalate salt.
m.p. = 97°C
MS(m/e): 497(M+1)
Calculated for C₃₁H₃₂N₂O₄-C₂H₂O₄: Theory: C, 67.57; H, 5.84; N, 4.78. Found: C, 67.76; H, 6.00; N, 4.85.

### EXAMPLE 37

### 1-((2S)-hydroxy-3-(naphth-2-yloxy)prop-1-yl)-4-hydroxy-4-(quinolin-3-yl)piperidine

Beginning with 0.132 gm (0.7 mMol) (S)-glycidyl naphth-2-yl ether and 0.150 gm (0.7 mMol) 4-hydroxy-4-(quinolin-3-yl)piperidine, 0.139 gm (49%) of the title compound were recovered as a white solid.
MS(m/e): 428(M⁺)
[a]_{D}²⁵(methanol) = -5.814°
Calculated for C₂₇H₂₈N₂O₃-0.25 H₂O Theory: C, 74.89; H, 6.63; N, 6.47. Found: C, 74.71; H, 6.67; N, 6.37.

### EXAMPLE 38

### 1-((2S)-hydroxy-3-(6-methoxynaph-2-yloxy)prop-1-yl)-4-hydroxy-4-(quinolin-3-yl)piperidine

Beginning with 0.151 gm (0.7 mMol) (S)-glycidyl 6-methoxynaphth-2-yl ether and 0.150 gm (0.7 mMol) 4-hydroxy-4-(quinolin-3-yl)piperidine, 0.172 gm (57%) of the title compound were recovered as a white foam. This foam was converted to the oxalate salt.
MS(m/e): 458(M⁺)
Calculated for C₂₈H₃₀N₂O₄-C₂H₂O₄: Theory: C, 65.68; H, 5.88; N, 5.11. Found: C, 65.43; H, 6.10; N, 5.06.

### EXAMPLE 39

### 1-((2S)-hydroxy-3-(indol-4-yloxy)prop-1-yl)-4-hydroxy-4-(quinolin-3-yl)piperidine

Beginning with 0.228 gm (1.2 mMol) (S)-glycidyl indol-4-yl ether and 0.275 gm (1.2 mMol) 4-hydroxy-4-(quinolin-3-yl)piperidine, 0.283 gm (56%) of the title compound were recovered as a white solid.
Calculated for C₂₅H₂₇N₃O₃: Theory: C, 71.92; H, 6.52; N, 10.06. Found: C, 71.76; H, 6.66; N, 9.99.
[a]_{D}²⁵(methanol) = +13.841°
This solid was converted to the oxalate salt.
m.p. = 117-120°C
MS(m/e): 417(M⁺)
[a]_{D}²⁵(methanol) = -7.018°

### EXAMPLE 40

### 1-((2S)-hydroxy-3-(indol-5-yloxy)prop-1-yl)-4-hydroxy-4-(quinolin-3-yl)piperidine

Beginning with 0.129 gm (0.7 mMol) (S)-glycidyl indol-5-yl ether and 0.156 gm (0.7 mMol) 4-hydroxy-4-(quinolin-3-yl)piperidine, 0.134 gm (47%) of the title compound were recovered as a white solid.
Calculated for C₂₅H₂₇N₃O₃: Theory: C, 71.92; H, 6.52; N, 10.06. Found: C, 71.63; H, 6.71; N, 9.85.
[a]_{D}²⁵(methanol) = +7.505°
This solid was converted to the oxalate salt.
m.p. = 122°C
MS(m/e): 417(M⁺)
Calculated for C₂₅H₂₇N₃O₃-C₂H₂O₄: Theory: C, 63.90; H, 5.76; N, 8.28. Found: C, 63.87; H, 5.76; N, 8.17.
[a]_{D}²⁵(methanol) = -6.479°

### EXAMPLE 41

### 1-((2R)-hydroxy-3-(indol-5-yloxy)prop-1-yl)-4-hydroxy-4-(quinolin-3-yl)piperidine

Beginning with 0.335 gm (1.8 mMol) (R)-glycidyl indol-5-yl ether and 0.404 gm (1.8 mMol) 4-hydroxy-4-(quinolin-3-yl)piperidine, 0.397 gm (54%) of the title compound were recovered as a white solid.
Calculated for C₂₅H₂₇N₃O₃-1.25 H₂O: Theory: C, 68.23; H, 6.18; N, 9.54. Found: C, 68.51; H, 6.28; N, 9.44.
[a]_{D}²⁵(methanol) = -12.635°
This solid was converted to the oxalate salt.
m.p. = 99°C
MS(m/e): 417(M⁺)
Calculated for C₂₅H₂₇N₃O₃-C₂H₂O₄: Theory: C, 63.90; H, 5.76; N, 8.28. Found: C, 64.40; H, 5.82; N, 8.36.
[a]_{D}²⁵(methanol) = +8.547°

### EXAMPLE 42

### 1-((2S)-hydroxy-3-(1-methylindol-4-yloxy)prop-1-yl)-4-hydroxy-4-(quinolin-3-yl)piperidine

Beginning with 0.134 gm (0.7 mMol) (S)-glycidyl 1-methylindol-4-yl ether and 0.150 gm (0.7 mMol) 4-hydroxy-4-(quinolin-3-yl)piperidine, 0.217 gm (76%) of the title compound were recovered as an off-white foam.
Calculated for C₂₆H₂₉N₃O₃-0.25 H₂O: Theory: C, 71.62; H, 6.82; N, 9.64. Found: C, 71.76; H, 6.82; N, 9.72.
[a]_{D}²⁵(methanol) = +22.414°
This foam was converted to the oxalate salt.
m.p. = 86°C
MS(m/e): 431(M⁺)
Calculated for C₂₆H₂₉N₃O₃-C₂H₂O₄: Theory: C, 64.48; H, 5.99; N, 8.06. Found: C, 64.21; H, 5.89; N, 7.95.
[a]_{D}²⁵(methanol) = -5.367°

### EXAMPLE 43

### 1-((2S)-hydroxy-3-(2-methylindol-4-yloxy)prop-1-yl)-4-hydroxy-4-(quinolin-3-yl)piperidine

Beginning with 0.134 gm (0.7 mMol) (S)-glycidyl 2-methylindol-4-yl ether and 0.150 gm (0.7 mMol) 4-hydroxy-4-(quinolin-3-yl)piperidine, 0.198 gm (68%) of the title compound were recovered as an off-white foam.
Calculated for C₂₆H₂₉N₃O₃-0.25 H₂O: Theory: C, 71.62; H, 6.82; N, 9.64. Found: C, 71.35; H, 6.60; N, 9.36.
[a]_{D}²⁵(methanol) = +3.766°
This foam was converted to the oxalate salt.
m.p. = 117°C
MS(m/e): 431(M⁺)
Calculated for C₂₆H₂₉N₃O₃-C₂H₂O₄: Theory: C, 64.48; H, 5.99; N, 8.06. Found: C, 64.33; H, 5.95; N, 8.06.
[a]_{D}²⁵(methanol) = -9.96°

### EXAMPLE 44

### 1-((2S)-hydroxy-3-(benzofur-4-yloxy)prop-1-yl)-4-hydroxy-4-(quinolin-3-yl)piperidine

Beginning with 0.094 gm (0.5 mMol) (S)-glycidyl benzofur-4-yl ether and 0.113 gm (0.5 mMol) 4-hydroxy-4-(quinolin-3-yl)piperidine, 0.088 gm (43%) of the title compound were recovered as an off-white foam. This foam was converted to the oxalate salt.
MS(m/e): 419(M+1)
Calculated for C₂₅H₂₆N₂O₄-C₂H₂O₄: Theory: C, 63.77; H, 5.55; N, 5.51. Found: C, 63.79; H, 5.66; N, 5.54.

### EXAMPLE 45

### 1-((2S)-hydroxy-3-(benzothien-4-yloxy)prop-1-yl)-4-hydroxy-4-(quinolin-3-yl)piperidine

Beginning with 0.090 gm (0.4 mMol) (S)-glycidyl benzothien-4-yl ether and 0.100 gm (0.4 mMol) 4-hydroxy-4-(quinolin-3-yl)piperidine, 0.139 gm (73%) of the title compound were recovered as an off-white foam. This foam was converted to the oxalate salt.
MS(m/e): 434(M⁺)
Calculated for C₂₅H₂₆N₂O₃S-C₂H₂O₄: Theory: C, 61.82; H, 5.38; N, 5.34. Found: C, 61.86; H, 5.21; N, 5.04.

### EXAMPLE 46

### 1-((2S)-hydroxy-3-(2-methylbenzothiazol-5-yloxy)prop-1-yl)-4-hydroxy-4-(quinolin-3-yl)piperidine

Beginning with 0.145 gm (0.7 mMol) (S)-glycidyl 2-methylbenzothiazol-5-yl ether and 0.150 gm (0.7 mMol) 4-hydroxy-4-(quinolin-3-yl)piperidine, 0.181 gm (61%) of the title compound were recovered as an off-white foam.
Calculated for C₂₅H₂₇N₃O₃S: Theory: C, 71.62; H, 6.82; N, 9.64. Found: C, 71.76; H, 6.82; N, 9.72.
[a]_{D}²⁵(methanol) = +22.414°
This foam was converted to the oxalate salt.
m.p. = 86°C
MS(m/e): 431(M⁺)
Calculated for C₂₆H₂₉N₃O₃-C₂H₂O₄: Theory: C, 64.48; H, 5.99; N, 8.06. Found: C, 64.21; H, 5.89; N, 7.95.
[a]_{D}²⁵(methanol) = -5.367°

### EXAMPLE 47

### 1-((2S)-hydroxy-3-(indan-4-yloxy)prop-1-yl)-4-hydroxy-4-(quinolin-3-yl)piperidine

Beginning with 0.150 gm (0.8 mMol) (S)-glycidyl indol-4-yl ether and 0.180 gm (0.8 mMol) 4-hydroxy-4-(quinolin-3-yl)piperidine, 0.202 gm (61%) of the title compound were recovered as a white foam.
Calculated for C₂₆H₃₀N₂O₃-0.25 H₂O: Theory: C, 73.82; H, 7.15; N, 6.62. Found: C, 73.66; H, 7.11; N, 6.92.
[a]_{D}²⁵(methanol) = +9.158°
This foam was converted to the oxalate salt.
m.p. = 85°C
MS(m/e): 418(M⁺)
[a]_{D}²⁵(methanol) = -5.254°

### EXAMPLE 48

### 1-((2S)-hydroxy-3-(indan-5-yloxy)prop-1-yl)-4-hydroxy-4-(quinolin-3-yl)piperidine

Beginning with 0.125 gm (0.7 mMol) (S)-glycidyl indol-5-yl ether and 0.150 gm (0.7 mMol) 4-hydroxy-4-(quinolin-3-yl)piperidine, 0.160 gm (58%) of the title compound were recovered as a white solid.
Calculated for C₂₆H₃₀N₂O₃-0.50 H₂O: Theory: C, 73.04; H, 7.31; N, 6.55. Found: C, 72.68; H, 7.13; N, 6.74.
[a]_{D}²⁵(methanol) = -205.31°
This solid was converted to the oxalate salt.
m.p. = 66°C
MS(m/e): 418(M⁺)
[a]_{D}²⁵(methanol) = -3.472°

### EXAMPLE 49

### 1-((2S)-hydroxy-3-(7-methylindan-4-yloxy)prop-1-yl)-4-hydroxy-4-(quinolin-3-yl)piperidine

Beginning with 0.134 gm (0.7 mMol) (S)-glycidyl 7-methylindan-4-yl ether and 0.150 gm (0.7 mMol) 4-hydroxy-4-(quinolin-3-yl)piperidine, 0.165 gm (58%) of the title compound were recovered as a light tan foam.
Calculated for C₂₇H₃₂N₂O₃: Theory: C, 74.97; H, 7.46; N, 6.48. Found: C, 74.81; H, 7.43; N, 6.18.
[a]_{D}²⁵(methanol) = +3.61°
This foam was converted to the oxalate salt.
m.p. = 94°C
MS(m/e): 432(M⁺)
Calculated for C₂₆H₂₉N₃O₃-C₂H₂O₄-0.25 H₂O: Theory: C, 66.08; H, 6.60; N, 5.31. Found: C, 65.87; H, 6.02; N, 5.46.
[a]_{D}²⁵(methanol) = -9.058°

### EXAMPLE 50

### 1-((2S)-hydroxy-3-(1,2,3,4-tetrahydronaphth-6-yloxy)prop-1-yl)-4-hydroxy-4-(quinolin-3-yl)piperidine

Beginning with 0.134 gm (0.7 mMol) (S)-glycidyl 1,2,3,4-tetrahydronaphth-6-yl ether and 0.150 gm (0.7 mMol) 4-hydroxy-4-(quinolin-3-yl)piperidine, 0.109 gm (38%) of the title compound were recovered as a white foam. This foam was converted to the oxalate salt.
MS(m/e): 433(M+1)
Calculated for C₂₇H₃₂N₂O₃-C₂H₂O₄: Theory: C, 66.65; H, 6.56; N, 5.36. Found: C, 66.51; H, 6.49; N, 5.64.

### EXAMPLE 51

### 1-((2S)-hydroxy-3-(quinolin-6-yloxy)prop-1-yl)-4-hydroxy-4-(quinolin-3-yl)piperidine

Beginning with 0.132 gm (0.7 mMol) (S)-glycidyl quinolin-6-yl ether and 0.150 gm (0.7 mMol) 4-hydroxy-4-(quinolin-3-yl)piperidine, 0.182 gm (65%) of the title compound were recovered as a white solid.
Calculated for C₂₆H₂₇N₃O₃-1.5 H₂O: Theory: C, 68.40; H, 6.62; N, 9.20. Found: C, 68.68; H, 6.17; N, 9.22.
[a]_{D}²⁵(methanol) = -24.39°
This solid was converted to the oxalate salt.
m.p. = 91°C
MS(m/e): 430(M+1)
Calculated for C₂₆H₂₇N₃O₃-C₂H₂O₄-H₂O: Theory: C, 62.56; H, 5.81; N, 7.82. Found: C, 62.66; H, 5.81; N, 7.58.
[a]_{D}²⁵(methanol) = -5.367°

### EXAMPLE 52

### 1-((2S)-hydroxy-3-(quinolin-7-yloxy)prop-1-yl)-4-hydroxy-4-(quinolin-3-yl)piperidine

Beginning with 0.132 gm (0.7 mMol) (S)-glycidyl quinolin-7-yl ether and 0.150 gm (0.7 mMol) 4-hydroxy-4-(quinolin-3-yl)piperidine, 0.199 gm (71%) of the title compound were recovered as an off-white foam. This foam was converted to the oxalate salt.
MS(m/e): 430(M+1)
Calculated for C₂₆H₂₇N₃O₃-C₂H₂O₄-H₂O: Theory: C, 62.56; H, 5.81; N, 7.82. Found: C, 62.76; H, 5.75; N, 7.53.

### EXAMPLE 53

### 1-((2S)-hydroxy-3-(julolidin-7-yloxy)prop-1-yl)-4-hydroxy-4-(quinolin-3-yl)piperidine

Beginning with 0.134 gm (0.5 mMol) (S)-glycidyl julolidin-7-yl ether and 0.125 gm (0.5 mMol) 4-hydroxy-4-(quinolin-3-yl)piperidine, 0.159 gm (61%) of the title compound were recovered as a light tan foam.
Calculated for C₂₉H₃₅N₃O₃-0.5 H₂O: Theory: C, 72.85; H, 7.48; N, 8.79. Found: C, 72.50; H, 7.15; N, 8.52.
[a]_{D}²⁵(methanol) = +7.59°
This foam was converted to the oxalate salt.
m.p. = 84°C
MS(m/e): 473(M⁺)
[a]_{D}²⁵(methanol) = -9.728°

### EXAMPLE 54

### 1-((2S)-hydroxy-3-(dibenzofur-3-yloxy)prop-1-yl)-4-hydroxy-4-(quinolin-3-yl)piperidine

Beginning with 0.202 gm (0.8 mMol) (S)-glycidyl dibenzofur-3-yl ether and 0.192 gm (0.8 mMol) 4-hydroxy-4-(quinolin-3-yl)piperidine, 0.133 gm (34%) of the title compound were recovered as an off-white foam.
Calculated for C₂₉H₂₈N₂O₄-0.5 H₂O: Theory: C, 72.94; H, 6.12; N, 5.87. Found: C, 73.01; H, 6.01; N, 5.87.
This foam was converted to the oxalate salt.
m.p. = 92°C
MS(m/e): 468(M⁺)
Calculated for C₂₉H₂₈N₂O₄-C₂H₂O₄-0.75 H₂O: Theory: C, 65.08; H, 5.55; N, 4.90. Found: C, 65.15; H, 5.59; N, 4.90.

### EXAMPLE 55

### 1-((2S)-hydroxy-3-(carbazol-2-yloxy)prop-1-yl)-4-hydroxy-4-(quinolin-3-yl)piperidine

Beginning with 0.157 gm (0.7 mMol) (S)-glycidyl carbazol-2-yl ether and 0.150 gm (0.7 mMol) 4-hydroxy-4-(quinolin-3-yl)piperidine, 0.034 gm (11%) of the title compound were recovered as a light tan solid.
MS(m/e): 467(M⁺)

### EXAMPLE 56

### 1-((2S)-hydroxy-3-(indol-4-yloxy)prop-1-yl)-4-hydroxy-4-(isoquinolin-4-yl)piperidine

Beginning with 0.166 gm (0.88 mMol) (S)-glycidyl indol-4-yl ether and 0.200 gm (0.88 mMol) 4-hydroxy-4-(isoquinolin-4-yl)piperidine, 0.125 gm (34%) of the title compound were recovered as a light yellow solid.
Calculated for C₂₅H₂₇N₃O₃-0.25 H₂O: Theory: C, 71.15; H, 6.45; N, 9.96. Found: C, 70.84; H, 6.55; N, 9.43.
[a]_{D}²⁵(methanol) = -7.233°
This solid was converted to the oxalate salt.
m.p. = 123°C
MS(m/e): 417(M⁺)
Calculated for C₂₅H₂₇N₃O₃-C₂H₂O₄-H₂O: Theory: C, 61.70; H, 5.56; N, 7.99. Found: C, 61.87; H, 5.55; N, 7.63.
[a]_{D}²⁵(methanol) = -18.382°

### EXAMPLE 57

### 1-((2S)-hydroxy-3-(indol-4-yloxy)prop-1-yl)-4-(pyridin-3-yl)piperidine

Beginning with 0.152 gm (0.8 mMol) (S)-glycidyl indol-4-yl ether and 0.130 gm (0.8 mMol) 4-(pyridin-3-yl)piperidine, 0.147 gm (52%) of the title compound were recovered as an off-white solid.
Calculated for C₂₁H₂₅N₃O₂-0.5 H₂O: Theory: C, 69.98; H, 6.99; N, 11.66. Found: C, 70.18; H, 6.89; N, 11.44.
[a]_{D}²⁵(methanol) = +12.195°
This solid was converted to the oxalate salt.
m.p. = 77°C
MS(m/e): 351(M⁺)
[a]_{D}²⁵(methanol) = -5.988°

### EXAMPLE 58

### 1-((2S)-hydroxy-3-(indol-4-yloxy)prop-1-yl)-4-(quinolin-3-yl)piperidine

Beginning with 0.133 gm (0.7 mMol) (S)-glycidyl indol-4-yl ether and 0.149 gm (0.7 mMol) 4-(quinolin-3-yl)piperidine, 0.133 gm (40%) of the title compound were recovered as an off-white solid.
Calculated for C₂₅H₂₇N₃O₂-0.25 H₂O: Theory: C, 73.96; H, 6.83; N, 10.35. Found: C, 73.72; H, 6.58; N, 10.16.
[a]_{D}²⁵(methanol) = +3.876°
This solid was converted to the oxalate salt.
m.p. = 124°C
MS(m/e): 401(M⁺)
[a]_{D}²⁵(methanol) = -7.207°

### EXAMPLE 59

### 1-((2S)-hydroxy-3-(indol-4-yloxy)prop-1-yl)-4-(isoquinolin-4-yl)piperidine

Beginning with 0.083 gm (0.4 mMol) (S)-glycidyl indol-4-yl ether and 0.093 gm (0.4 mMol) 4-(isoquinolin-4-yl)piperidine, 0.072 gm (41%) of the title compound were recovered as an off-white solid. This solid was converted to the oxalate salt.
m.p. = 118°C
MS(m/e): 401(M⁺)
Calculated for C₂₅H₂₇N₃O₂-C₂H₂O₄-H₂O: Theory: C, 63.64; H, 5.74; N, 8.25. Found: C, 63.77; H, 5.62; N, 8.25.
[a]_{D}²⁵(methanol) = -15.2381°

### EXAMPLE 60

### 1-((2S)-hydroxy-3-(indol-4-yloxy)prop-1-yl)-4-(quinoxalin-2-yl)piperidine

Beginning with 0.108 gm (0.6 mMol) (S)-glycidyl indol-4-yl ether and 0.122 gm (0.6 mMol) 4-(quinoxalin-2-yl)piperidine, 0.137 gm (60%) of the title compound were recovered as a light tan foam. This foam was converted to the oxalate salt.
MS(m/e): 402(M⁺)
Calculated for C₂₄H₂₆N₄O₂-C₂H₂O₄: Theory: C, 63.40; H, 5.73; N, 11.38. Found: C, 63.30; H, 5.85; N, 11.15.

Compounds and compositions useful for the method of the present invention must both have affinity for and be antagonists at the 5-HT_{1F} receptor. The ability of compounds useful for the method of this invention to bind to the 5-HT_{1F} receptor subtype is measured essentially as described in N. Adham, *et al., Proceedings of the National Academy of Sciences (USA),* **90**, 408-412 (1993).

Membrane Preparation: Membranes were prepared from transfected Ltk- cells which were grown to 100% confluency. The cells were washed twice with phosphate-buffered saline, scraped from the culture dishes into 5 mL of ice-cold phosphate-buffered saline, and centrifuged at 200 x g for 5 minutes at 4°C. The pellet was resuspended in 2.5 mL of ice-cold Tris buffer (20 mM Tris HCl, pH=7.4 at 23°C, 5 mM EDTA) and homogenized with a Wheaton tissue grinder. The lysate was subsequently centrifuged at 200 x g for 5 minutes at 4°C to pellet large fragments which were discarded. The supernatant was collected and centrifuged at 40,000 x g for 20 minutes at 4°C. The pellet resulting from this centrifugation was washed once in ice-cold Tris wash buffer and resuspended in a final buffer containing 50 mM Tris HCl and 0.5 mM EDTA, pH=7.4 at 23°C. Membrane preparations were kept on ice and utilized within two hours for the radioligand binding assays. Protein concentrations were determined by the method of Bradford (*Anal. Biochem.,* **72**, 248-254 (1976)).

Radioligand Binding: [³H-5-HT] binding was performed using slight modifications of the 5-HT_{1D} assay conditions reported by Herrick-Davis and Titeler (*J. Neurochem.,* **50**, 1624-1631 (1988)) with the omission of masking ligands. Radioligand binding studies were achieved at 37°C in a total volume of 250 mL of buffer (50 mM Tris, 10 mM MgCl₂, 0.2 mM EDTA, 10 mM pargyline, 0.1% ascorbate, pH=7.4 at 37°C) in 96 well microtiter plates. Saturation studies were conducted using [³H]5-HT at 12 different concentrations ranging from 0.5 nM to 100 nM. Displacement studies were performed using 4.5-5.5 nM [³H]5-HT. The binding profile of drugs in competition experiments was accomplished using 10-12 concentrations of compound. Incubation times were 30 minutes for both saturation and displacement studies based upon initial investigations which determined equilibrium binding conditions. Nonspecific binding was defined in the presence of 10 mM 5-HT. Binding was initiated by the addition of 50 mL membrane homogenates (10-20 µg), The reaction was terminated by rapid filtration through presoaked (0.5% poylethyleneimine) filters using 48R Cell Brandel Harvester (Gaithersburg, MD). Subsequently, filters were washed for 5 seconds with ice cold buffer (50 mM Tris HCl, pH=7.4 at 4°C), dried and placed into vials containing 2.5 mL Readi-Safe (Beckman, Fullerton, CA) and radioactivity was measured using a Beckman LS 5000TA liquid scintillation counter. The efficiency of counting of [³H]5-HT averages between 45-50%. Binding data was analyzed by computer-assisted nonlinear regression analysis (Accufit and Accucomp, Lunden Software, Chagrin Falls, OH). IC₅₀ values were converted to Kᵢ values using the Cheng-Prusoff equation (*Biochem. Pharmacol.,* **22**, 3099-3108 (1973). All experiments were performed in triplicate. Representative compounds of Formula I were tested in this assay and were found to have affinity at the 5-HT_{1F} receptor.

As was reported by R.L. Weinshank, *et al.,* WO93/14201, the 5-HT_{1F} receptor is functionally coupled to a G-protein as measured by the ability of serotonin and serotonergic drugs to inhibit forskolin stimulated cAMP production in NIH3T3 cells transfected with the 5-HT_{1F} receptor. Agonist activation of G-protein-coupled receptors also results in the release of GDP from the α-subunit of the G protein and the subsequent binding of GTP. The binding of the stable analog [³⁵S]GTPγS is an indicator of this receptor activation. In vitro 5-HT_{1F} receptor activation, as measured by [³⁵S]GTPγS binding, was carried out essentially as described by Wainscott, *et al., European Journal of Pharmacology*, **352**, 117-124 (1998).

### Membrane Preparation

Mouse LM(tk-)cells stably transfected with the human 5-HT_{1F} receptor and grown in suspension were harvested by centrifugation, resuspended in 50 mM Tris-HCl, pH 7.4, in aliquots of 2 x 10⁸ cells and frozen at -70°C until the day of the assay. On the assay day, an aliquot of cells was thawed, resuspended in 35 mL of 50 mM Tris-HCl, pH 7.4, and centrifuged at 39,800 x g for 10 minutes at 4°C. The resulting pellet was resuspended in 50 mM Tris-HCl, pH 7.4, incubated for 10 minutes at 37°C and centrifuged at 39,800 x g for 10 minutes at 4°C. The pellet was resuspended and centrifuged once more, with the final pellet being resuspended in 4 mM MgCl₂, 160 mM NaCl, 0.267 mM EGTA, 67 mM Tris-HCl, pH 7.4, such that a 200 mL aliquot contained contained approximately 15-25 mg protein.

### [³⁵S]GTPγS binding

The assay was modified from published conditions (Sim *et al., Proc. Natl. Acad. Sci. USA,* **92**, 7242-7246 (1995); Thomas *et al., J. Rec. Signal Transduct. Res.,* **15**, 199-211 (1995)). Two versions of the assay, one using vacuum filtration (Wainscott *et al., Eur J. Pharmacol.,* **352**, 117-124 (1998)) and one using a scintillation proximity assay, have been developed for the determination of the potency and intrinsic activity (efficacy) of 5-HT_{1F} ligands.

### [³⁵S]GTPγS binding using vacuum filtration

Incubations were performed in a total volume of 800 µl. Test compounds in water (glacial acetic acid and/or dimethyl sufoxide [DMSO] may have been used to aid in solubilizing some compounds), 200 µl, were added to 400 µl of Tris-HCl, pH 7.4, containing MgCl₂, NaCl, EGTA, GDP and [³⁵S]GTPγS. Membrane homogenate (200 µl) was added and the tubes were incubated for 30 min at 37°C. The final concentrations of MgCl₂, NaCl, EGTA, GDP, [³⁵S]GTPγS and Tris were 3 mM, 120 mM, 0.2 mM, 10 µM, 0.1 nM and 50 mM, respectively. For experiments examining the inhibition of 5-HT stimulated [³⁵S]GTPγS binding, the final concentration of 5-HT was 1 µM. Using a Brandel cell harvester (model MB-48R, Brandel, Gaithersburg, MD), the incubations were terminated by vacuum filtration through Whatman GF/B filters which had been wetted with water or 20 mM Na₄P₂O₇ and pre-cooled with 4 ml of ice-cold 50 mM Tris-HCl, pH 7.4. The filters were then rapidly washed with 4 ml of ice-cold 50 mM Tris-HCl, pH 7.4. The amount of [³⁵S]GTPγS captured on the filters was determined by liquid scintillation spectrometry.

### [³⁵S]GTPγS binding using a scintillation proximity assay

Incubations were performed in a total volume of 200 µl in 96 well assay plates. [³⁵S]GTPγS and guanosine-5'-diphosphate in assay buffer (MgCl₂, NaCl, EGTA in Tris-HCl, pH 7.4), 50 µl, were added to 50 µl of test compounds dissolved in water (glacial acetic acid and/or dimethyl sufoxide [DMSO] may have been used to aid in solubilizing some compounds). Wheat Germ Agglutinin (WGA) beads (Amersham Life Sciences, Inc., Arlington Heights, IL) for scintillation proximity assay (SPA), in assay buffer (20, 25 or 50 µl) were then added. Membrane homogenate (80, 75 or 50 µl) was added and the plates were covered with sealing tape (Wallac, Inc., Gaithersburg, MD), and allowed to incubate at room temperature for 2 hr. The final concentrations of MgCl₂, NaCl, EGTA, GDP, [³⁵S]GTPγS and Tris were 3 mM, 120 mM, 0.2 mM, 10 µM, 0.25 nM and 50 mM, respectively. For experiments examining the inhibition of 5-HT-stimulated [³⁵S]GTPγS binding, the final concentration of 5-HT was 1 µM. The plates were then centrifuged at approximately 200 x *g* for 10 min at room temperature. The amount of [³⁵S]GTPγS bound to the membranes, i.e., in close proximity to the WGA SPA beads, was then determined using a Wallac MicroBeta® Trilux Scintillation Counter (Wallac, Inc.).

### Data Analysis

Nonlinear regression analysis was performed on the concentration-response curves (generating EC₅₀ and Eₘₐₓ values for stimulation of [³⁵S]GTPγS binding or generating IC₅₀ and Eₘₐₓ values for inhibition of 5-HT-stimulated [³⁵S]GTPγS binding) using a four parameter logistic equation described by DeLean et al. (De Lean *et al., Mol. Pharmacol.,* **21**, 5-16 (1982)). The equation was modified such that the slope was a positive number for stimulation of [³⁵S]GTPγS binding. Efficacy (Eₘₐₓ) values, determined by the nonlinear regression analysis, for selected compounds were expressed as the percent of [³⁵S]GTPγS binding relative to 10 µM 5-HT which was run as a standard with each concentration-response curve. Efficacy values for compounds run as single point determinations may have also been calculated relative to 10 µM 5-HT-stimulated [³⁵S]GTPγS binding, which was run as a standard on each of these assay plates. For inhibition of 5-HT-stimulated [³⁵S]GTPγS binding, the IC₅₀ values were converted to Kᵢ values using the Cheng-Prusoff equation (Cheng and Prusoff, *Biochem. Pharmacol.,* **22**, 3099-3108 (1973)). In addition, the minimum for inhibition of 5-HT-stimulated [³⁵S]GTPγS binding also represents a determination of the efficacy (Eₘₐₓ) value for the tested compounds, calculated as the percent of [³⁵S]GTPγS binding relative to 10 µM 5-HT alone as the standard run with each of these curves.

The utility of serotonin 5-HT_{1F} antagonists for the treatment of anxiety disorders was demonstrated by the social interaction assay described by T.J. Sajdyk and A. Shekhar in *The Journal of Pharmacology and Experimental Therapeutics,* **283,** 969-977 (1997), a fully validated test of anxiety (S.E. File, *Journal of Neuroscience Methods,* **2**, 219-238 (1980)). The procedure for this assay is summarized below.

### Animals

All experiments were performed on male Wister rats (Harlan laboratories, 275-300 gm). The rats were individually housed in a temperature controlled room (72°F) on a 12 hour day/night cycle. The rats were provided food and water ad libitum.

### Test Compound

Approximately 40 mg of the test compound was weighed into a 20 mL glass vial. To this solid were added 80µL of lactic acid (85%). The mixture was sonicated for 2 minutes and then 4mL of distilled water was added in lmL increments. Sonication of this mixture was continued until all of the solid had dissolved. Following sonication, the pH of the mixture was raised by the addition of dilute aqueous sodium hydroxide until the mixture reached a pH of about 5.5.

### Protocols

Each rat was weighed and the test compound administered by intraperitoneal injection at 20 mg/kg of body weight. The animals were then placed back in their home cages for 1 hour before the behavioral protocol was initiated. Each animal was first tested for baseline activity in the assay, and then was tested two days later with compound.

Experimental anxiety was measured by a social interaction test. The apparatus was a solid wood box (36 inches long x 36 inches wide x 12 inches high) with an open roof. A video camera was fixed above the social interaction box and all behavioral tests were recorded. During the test session, the rat which received the test compound was placed into the social interaction box with another male Wistar rat which had been housed individually and was unfamiliar to the rat which received the test compound. The rat which received the test compound was observed for 5 minutes. The amount of time that the rat which received test compound spent interacting (i.e. grooming, sniffing, crawling upon) with the other rat was recorded. All tests were performed under lighted conditions. An increase in interaction time represents a decrease in anxiety. Conversely, a decrease in interaction time represents an increase in anxiety.

### Statistical Analysis

Social interaction data was analyzed as total interaction time in seconds and the raw scores were compared between baseline and treatment. Statistical analyses were conducted by using a paired students t-test. Statistical significance was accepted at p<0.01.

The serotonin 5-HT_{1F} antagonist of Example 37 was tested in this assay and was found to significantly increase social interaction time as compared to baseline at a dose of 20 mg/kg.

The suitability of a compound or composition for use in the method of the present invention, therefore, is determined as follows:
1. Demonstration of affinity for the 5-HT_{1F} receptor; and
2. Once affinity for the 5-HT_{1F} receptor is established, determination of full antagonist, partial antagonist, or inverse agonist activity.

Anxiety disorders are a heterogeneous class of diseases. The most common types of anxiety disorders are described in the following paragraphs.

### Panic Disorder

Panic disorder is characterized by the sudden onset of intense apprehension, fearfulness, or terror. An attack of panic disorder is unprovoked and may last for a discrete period of time. During these attacks, it is not uncommon for the victim to experience shortness of breath, palpitations, chest pain or discomfort, choking or a smothering sensation, and fear of losing control.

### Generalized Anxiety Disorder

Generalized anxiety disorder is characterized by at least 6 months of persistent and excessive anxiety and worry. It is associated with physical anxiety symptoms such as muscle aches, fatigue, difficulty sleeping, sweating, dizziness, and nausea.

### Specific Phobia

Specific phobia is a persistent, intense, and irrational fear associated with a particular object or situation that leads to avoidance of that object or situation.

### Social Phobia

Social phobia is a persistent fear of one or more situations in which the person is exposed to possible scutiny by others and the person fears that he or she may do something or act in a way that will be humiliating. Social phobias can include extreme shyness.

### Obsessive-Compulsive Disorder

Obsessive-compulsive disorder is characterized by obsessions which cause anxiety and compulsions which serve to neutralize the anxiety. Common obsessions include fear of dirt, germs, or contamination or fear of harming someone; common compulsions are excessive cleaning, counting, double-checking, and hoarding.

### Post-Traumatic Stress Disorder

Post-traumatic stress disorder is characterized by the reexperiencing of an extremely traumatic event accompanied by symptoms of increased arousal and by avoidance of the stimuli associated with the trauma. Individuals can become so preoccupied with the experience that they are unable to lead a normal life.

The diseases described above as well as other anxiety disorders contemplated by the method of the present invention are classified in the Diagnostic and Statistical Manual of Mental Disorders, 4th Version, published by the American Psychiatric Association (DSM). In such cases, the DSM code numbers are supplied below for the convenience of the reader.

| | |
|---|---|
| Panic Disorder Without Agoraphobia | DSM 300.01 |
| Panic Disorder With Agoraphobia | DSM 300.21 |
| Agoraphobia Without History of Panic Disorder | DSM 300.22 |
| Specific Phobia | DSM 300.29 |
| Social Phobia | DSM 300.23 |
| Obsessive-Compulsive Disorder | DSM 300.3 |
| Post-Traumatic Stress Disorder | DSM 309.81 |
| Acute Stress Disorder | DSM 308.3 |
| Generalized Anxiety Disorder | DSM 300.02 |
| Anxiety Disorder Due to a General Medical | |
| Condition | DSM 293.84 |
| Substance Induced Anxiety Disorder | |
| Alcohol | DSM 291.89 |
| Amphetamine (or Amphetamine-Like Substance) | DSM 292.89 |
| Caffeine | DSM 292.89 |
| Cannabis | DSM 292.89 |
| Cocaine | DSM 292.89 |
| Hallucinogen | DSM 292.89 |
| Inhalant | DSM 292.89 |
| Phencyclidine (or Phencyclidine-Like Substance) | DSM 292.89 |
| Sedative, Hypnotic, or Anxiolytic | DSM 292.89 |
| Other [Unknown] Substance | DSM 292.89 |
| Anxiety Disorder Not Otherwise Specified | DSM 300.00 |
| Separation Anxiety Disorder | DSM 309.21 |
| Sexual Adversion Disorder | DSM 302.79 |

Any of these disorders, whether presenting alone or in combination in an individual mammal, may be treated or prevented by the method of the present invention.

While it is possible to administer a compound employed in the methods of this invention directly without any formulation, the compounds are usually administered in the form of pharmaceutical compositions comprising a pharmaceutically acceptable excipient and at least one active ingredient. These compositions can be administered by a variety of routes including oral, rectal, transdermal, subcutaneous, intravenous, intramuscular, and intranasal. Many of the compounds employed in the methods of this invention are effective as both injectable and oral compositions. Such compositions are prepared in a manner well known in the pharmaceutical art and comprise at least one active compound. See, e.g., REMINGTON'S PHARMACEUTICAL SCIENCES, (16th ed. 1980).

In making the compositions employed in the present invention the active ingredient is usually mixed with an excipient, diluted by an excipient or enclosed within such a carrier which can be in the form of a capsule, sachet, paper or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing for example up to 10% by weight of the active compound, soft and hard gelatin capsules, suppositories, sterile injectable solutions, and sterile packaged powders.

In preparing a formulation, it may be necessary to mill the active compound to provide the appropriate particle size prior to combining with the other ingredients. If the active compound is substantially insoluble, it ordinarily is milled to a particle size of less than 200 mesh. If the active compound is substantially water soluble, the particle size is normally adjusted by milling to provide a substantially uniform distribution in the formulation, e.g. about 40 mesh.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and methyl cellulose. The formulations can additionally include: lubricating agents such as talc, magnesium stearate, and mineral oil; wetting agents; emulsifying and suspending agents; preserving agents such as methyl- and propylhydroxybenzoates; sweetening agents; and flavoring agents. The compositions of the invention can be formulated so as to provide quick, sustained or delayed release of the active ingredient after administration to the patient by employing procedures known in the art.

The compositions are preferably formulated in a unit dosage form, each dosage containing from about 0.05 to about 100 mg, more usually about 1.0 to about 30 mg, of the active ingredient. The term "unit dosage form" refers to physically discrete units suitable as unitary dosages for human subjects and other mammals, each unit containing a predetermined quantity of active material calculated to produce the desired therapeutic effect, in association with a suitable pharmaceutical excipient.

The active compounds are generally effective over a wide dosage range. For examples, dosages per day normally fall within the range of about 0.01 to about 30 mg/kg of body weight. In the treatment of adult humans, the range of about 0.1 to about 15 mg/kg/day, in single or divided dose, is especially preferred. However, it will be understood that the amount of the compound actually administered will be determined by a physician, in the light of the relevant circumstances, including the condition to be treated, the chosen route of administration, the actual compound or compounds administered, the age, weight, and response of the individual patient, and the severity of the patient's symptoms, and therefore the above dosage ranges are not intended to limit the scope of the invention in any way. In some instances dosage levels below the lower limit of the aforesaid range may be more than adequate, while in other cases still larger doses may be employed without causing any harmful side effect, provided that such larger doses are first divided into several smaller doses for administration throughout the day.

### Formulation Example 1

Hard gelatin capsules containing the following ingredients are prepared:

| Ingredient | Quantity (mg/capsule) |
|---|---|
| Compound of Example 37 | 30.0 |
| Starch | 305.0 |
| Magnesium stearate | 5.0 |

The above ingredients are mixed and filled into hard gelatin capsules in 340 mg quantities.

### Formulation Example 2

A tablet formula is prepared using the ingredients below:

| Ingredient | Quantity (mg/tablet) |
|---|---|
| Compound of Example 37 | 25.0 |
| Cellulose, microcrystalline | 200.0 |
| Colloidal silicon dioxide | 10.0 |
| Stearic acid | 5.0 |

The components are blended and compressed to form tablets, each weighing 240 mg.

Another preferred formulation employed in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art. See, e.g., U.S. Patent 5,023,252, issued June 11, 1991, herein incorporated by reference. Such patches may be constructed for continuous, pulsatile, or on demand delivery of pharmaceutical agents.

Frequently, it will be desirable or necessary to introduce the pharmaceutical composition to the brain, either directly or indirectly. Direct techniques usually involve placement of a drug delivery catheter into the host's ventricular system to bypass the blood-brain barrier. One such implantable delivery system, used for the transport of biological factors to specific anatomical regions of the body, is described in U.S. Patent 5,011,472, issued April 30, 1991, which is herein incorporated by reference.

Indirect techniques, which are generally preferred, usually involve formulating the compositions to provide for drug latentiation by the conversion of hydrophilic drugs into lipid-soluble drugs or prodrugs. Latentiation is generally achieved through blocking of the hydroxy, carbonyl, sulfate, and primary amine groups present on the drug to render the drug more lipid soluble and amenable to transportation across the blood-brain barrier. Alternatively, the delivery of hydrophilic drugs may be enhanced by intra-arterial infusion of hypertonic solutions which can transiently open the blood-brain barrier.

The type of formulation employed for the administration of the compounds employed in the methods of the present invention may be dictated by the particular compounds employed, the type of pharmacokinetic profile desired from the route of administration and the compound(s), and the state of the patient.

## Claims

1. The use of a serotonin 5-HT_{1F} antagonist in the manufacture of a medicament for the treatment or prevention of anxiety disorders.

2. The use of a full antagonist of the serotonin 5-HT_{1F} receptor in the manufacture of a medicament for the treatment or prevention of anxiety disorders.

3. The use of a partial antagonist of the serotonin 5-HT_{1F} receptor in the manufacture of a medicament for the treatment or prevention of anxiety disorders.

4. A method for the treatment or prevention of anxiety disorders, comprising administering to a mammal in need such treatment an effective amount of a serotonin 5-HT_{1F} antagonist.

5. A method for the treatment or prevention of anxiety disorders, comprising administering to a mammal in need of such treatment an effective amount of a full antagonist of the serotonin 5-HT_{1F} receptor.

6. A method for the treatment or prevention of anxiety disorders, comprising administering to a mammal in need of such treatment an effective amount of a partial antagonist of the serotonin 5-HT_{1F} receptor.

7. A method of any of Claims 4-6, where the antagonist of the serotonin 5-HT_{1F} receptor is selective for the serotonin 5-HT_{1F} receptor relative to other serotonin receptors.

8. A method of any of Claims 4-7, where the anxiety disorder is selected from the group consisting of panic disorder, generalized anxiety disorder, specific phobia, social phobia, obsessive-compulsive disorder, and post-traumatic stress disorder.

9. A method of any of Claims 4-8 where the mammal is a human.
